# EUROPÄISCHE PATENTANMELDUNG

(11) **EP 2 918 271 A1**
(43) Veröffentlichungstag der Anmeldung: **16.09.2015**
(21) Anmeldenummer: 14170001.3
(22) Anmeldetag: 27.05.2014
(51) Int. Cl.: A61K 31/36, C07C 57/44, C07D 317/64

(54) **Aromatische Alkensäurederivate als Würzstoffe**

(30) Priorität: 12.03.2014 EP 14159306
(71) Anmelder: Symrise AG, 37603 Holzminden (DE)
(72) Erfinder: Ley, Jakob, 37603 Holzminden (DE); Reichert, Katharina, 37603 Holzminden (DE); Obst, Katja, 37603 Holzminden (DE)
(74) Vertreter: Fabry, Bernd

(57) **Zusammenfassung**

Die Erfindung befindet sich auf dem Gebiet der Geschmacksstoffe und betrifft insbesondere die Verwendung von aromatischen Alkensäurederivaten der Formel (I) als Geschmacksstoffe sowie Aromamischungen und orale Zubereitungen, die diese Amide enthalten.

## Beschreibung

### GEBIET DER ERFINDUNG

Die Erfindung befindet sich auf dem Gebiet der Geschmacksstoffe und betrifft insbesondere die Verwendung von aromatischen Alkensäurederivaten der Formel (I) als Geschmacksstoffe sowie Aromamischungen und orale Zubereitungen, die diese Amide enthalten.

### STAND DER TECHNIK

Es besteht ein ständiger Bedarf, neue Geschmacksstoffe, d.h. geschmacksaktive Verbindungen bzw. Verbindungen, die einen Geschmackseindruck vermitteln, modifizieren und/oder verstärken können, aufzufinden. Insbesondere besteht Bedarf an solchen Verbindungen, die den Geschmackseindruck Umami und/oder Kokumi vermitteln bzw. auf den Geschmacksknospen erzeugen, modifizieren und/oder verstärken können. Im Zuge des verstärkten Gesundheitsbewusstseins der Konsumenten werden zudem Verbindungen gesucht, die einen salzigen Geschmackseindruck vermitteln, modifizieren und/oder verstärken können. Insgesamt besteht demnach ein besonderer Bedarf an würzigen Geschmacksstoffen, die sämtliche der Geschmackseindrücke Umami, Kokumi und salzig vermitteln, modifizieren und/oder verstärken können. Besonders bevorzugt sind im allgemeinen Geschmacksstoffe, die in natürlichen, frischen, getrockneten oder gegebenenfalls mit für Lebensmittel üblichen Zubereitungsarten (z.B. Evaporation oder Pervaporation, Extraktion, Dämpfen, Erhitzen, Rösten, Kochen, Braten, Kühlen, Mahlen, Enzym-Behandlung, Fermentieren) behandelten Quellen tierischen, pflanzlichen, pilzlichen oder mikrobiellen Ursprungs gefunden und im Idealfall aus diesen isoliert werden können ("natürlich vorkommende" Stoffe).

Unter einem würzigen Geschmackseindruck versteht man insbesondere den als Umami bezeichneten Geschmack der Aminosäuren Glutaminsäure und Asparaginsäure und der Nucleotide Adenosin-5'-monophosphat, Cytidin-5'-monophosphat, Inosin-5'-monophosphat, Guanosin-5'-monophosphat, insbesondere in der Form ihrer Mononatriumsalze, insbesondere auch in Mischungen der vorgenannten Stoffe, wobei der Umami-Geschmack auch durch weitere, hier nicht aufgeführte Verbindungen verursacht werden kann. Der Geschmackseindruck Umami wird häufig mit den Begriffen "Brühe-artig", "fleischig", "mundfüllend" und "würzig" umschrieben und häufig im Zusammenhang mit dem Geschmackseindruck Kokumi gesehen. Zudem trägt der Umami-Geschmackseindruck häufig im Rahmen der Gesamtgeschmackswahrnehmung zur Salzigkeit bei, obwohl Salzigkeit insbesondere durch Natriumionen, vor allem in Form von Natriumchlorid, hervorgerufen wird. Die vorstehend genannten Aminosäuren und Nucleotide bzw. Salze haben den Nachteil, dass eine relativ hohe Konzentration dieser Stoffe eingesetzt werden muss, um einen zufriedenstellenden Umami- bzw. Kokumi-Geschmackseindruck zu vermitteln. So muss beispielsweise Natriumgluatamat regelmäßig in einer Konzentration von 0,02 bis 0,5 Gew.-%, bezogen auf das Gesamtgewicht des Lebensmittels, vorhanden sein, um den gewünschten Geschmackseindruck zu erzeugen. Die vorstehend genannten Nucleotide sind zudem für sich alleine jeweils nur schwach wirksam und können daher häufig nur mit Natriumglutamat zusammen einen zufriedenstellenden Umami-Geschmackseindruck vermitteln.

Erst in den letzten Jahren sind einige nicht natürlich vorkommende Verbindungen mit einer wesentlich potenteren Umami-ähnlichen Wirkung beschrieben worden, z. B. in den Schriften EP 1989944 A1**,** WO 2008 046895 A1**,** EP 2168442 A2 (alle Symrise), US 2004 0202760 A1**,** US 2006 057268 und US 2007 0134389 (alle IFF). Diese Verbindungen besitzen häufig eine 10- bis 10000-fach stärkere Geschmacksstoffwirkung als Mononatriumglutamat.

Durch molekularbiologische Methoden ist es außerdem gelungen, den wesentlichen Rezeptor des Menschen, der für den Glutamat- und Umami-Geschmack zuständig ist, zu identifizieren. Basierend auf diesen Erkenntnissen wurden in WO 2003 004992 A2 (NIH) und WO 2003 001876 A2 (Senomyx) Messmethoden vorgeschlagen, um neue möglicherweise sensorisch wirksame Umami-Geschmacksstoffe zu identifizieren. So werden in der Veröffentlichung US 2005 084506 A (Senomyx) einige potentielle Aktivatoren des Umami-Rezeptors in Form nicht natürlich vorkommender Amide beschrieben. Aus den genannten Schriften sind beispielsweise einige nicht natürlich vorkommende Zimtamide aromatischer Amine beschrieben, die den fremdexprimierten humanen, potentiellen Umami-Rezeptor aktivieren können.

Die vorstehend genannten Verbindungen sind nicht natürlich vorkommende Verbindungen. Generell - wie auch im Rahmen der vorliegenden Erfindung - sind jedoch natürlich vorkommende Verbindungen gegenüber synthetischen bzw. nicht-natürlich vorkommenden Verbindungen bevorzugt. Natürlich vorkommende, einen potenten Umami-Geschmackseindruck vermittelnde oder verstärkende Amide vorstehend genannter Strukturtypen wurden im Stand derTechnik bisher jedoch nicht beschrieben.

In der Veröffentlichung EP 2,529,632 B1 (Symrise) wird die Verwendung bestimmter natürlich vorkommender Zimtsäureamide aromatischer Amine als Aromastoffe und Umamiverbindungen beschrieben. Die stärkste Wirkung haben dabei Rubemamin und Rubescenamin. Es ist aus der Schrift nicht trivial abzuleiten, dass alle Zimtsäureamide generell wirksam sind. So ist das als Scharfstoff bekannte und strukturell verwandte aromatische Alkenamid Piperin (vgl. folgende Strukturformel) nicht Umami-wirksam.

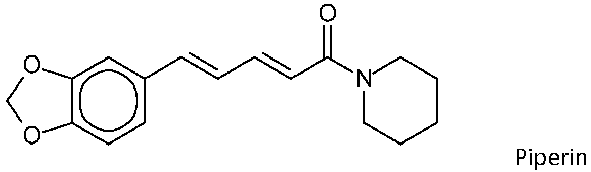

Die Aufgabe der vorliegenden Erfindung hat somit darin bestanden, Verbindungen und Stoffe zu finden, die als Geschmackstoffe eingesetzt werden können. Insbesondere war es Aufgabe der Erfindung, Verbindungen und Stoffe zu finden, die als Geschmackstoffe einen Beitrag zur Vermittelung, Modifizierung und/oder Verstärkung von Geschmackseindrücken, insbesondere im Bereich Umami, Kokumi und salzig, aufweisen. Ferner war es Aufgabe der vorliegenden Erfindung, Stoffe und Verbindungen zu finden, die nicht toxisch oder nur beschränkt einsetzbar sind, so dass sie im Lebensmittelbereich eingesetzt werden können. Vor allem sollten die neuen Geschmackstoffe bzw. -verbindungen bereits in geringen Mengen wirken und trotzdem eine ca. 10-fach stärkere Geschmacksstoffwirkung als Natriumglutamat besitzen und dabei einen besonders sauberen Geschmackseindruck vermitteln, d.h. einen Geschmackseindruck, der nicht durch weitere Geschmacksrichtungen verfälscht oder beeinflusst wird. Zudem sollten diese Geschmackstoffe bzw. -verbindungen möglichst keine unangenehmen sensorischen Noten oder einen negativen Nebengeschmack in Produkten aufweisen. Des Weiteren galt es, die gefundenen neuen Geschmacksstoffe in Nahrungsmittel einzuarbeiten, insbesondere in Zubereitungen, die der Ernährung oder dem Genuss dienende (gebrauchs- oder verzehrfertige) Zubereitungen und Halbfertigwaren. Hierbei sind insbesondere Nahrungsmittel und Zubereitungen, die Natriumglutamat-, Salz- und Zucker-reduziert oder-frei sind, besonders bevorzugt.

### BESCHREIBUNG DER ERFINDUNG

Die Aufgaben konnten mit den in der vorliegenden Erfindung gefundenen Verbindungen bzw. Stoffe gelöst werden. Gegenstand der Erfindung sind daher aromatische Alkensäurederivate der Formel (I) 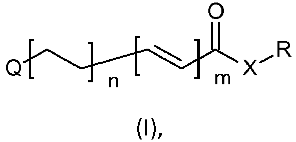 wobei Q einen Rest 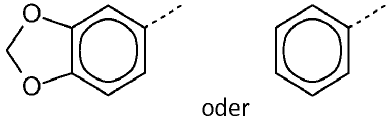 darstellt,
und entweder
i) n einen Wert 0 und gleichzeitig m einen Wert 1
   oder
ii) n einen Wert 1 und m gleichzeitig einen Wert 1 oder 2 darstellt,
   und
wobei die resultierende(n) Doppelbindung(en) unabhängig voneinander als E-oder Z-Konfiguration vorliegen,
und
X = - OR¹ oder - NR²R³ bedeutet, mit
R¹ = Wasserstoff, Methyl, Ethyl, Propyl, 2-Propyl, 1-Butyl, 2-Butyl, 2-Methyl-1-propyl, 1-Pentyl, 2-Pentyl, 3-Pentyl, 2-Methyl-1-butyl, 2-Methyl-2-butyl, 2-Methyl-3-butyl, 3-Methyl-1-butyl, 1-Hexyl, 2-Hexyl, 3-Hexyl, 2-Methyl-1-pentyl, 2-Methyl-2-pentyl, 2-Methyl-3-pentyl, 2-Methyl-4-pentyl, 3-Methyl-1-pentyl, 3-Methyl-2-pentyl oder 3-Methyl-3-pentyl, Allyl, Prenyl oder Isoprenyl,
   und wobei
R², R³ unabhängig voneinander ausgewählt ist aus der Gruppe bestehend aus: Wasserstoff, Methyl, Ethyl, Propyl, 2-Propyl, 1-Butyl, 2-Butyl, 2-Methyl-1-propyl, 1-Pentyl, 2-Pentyl, 3-Pentyl, 2-Methyl-1-butyl, 2-Methyl-2-butyl, 2-Methyl-3-butyl, 3-Methyl-1-butyl, 1-Hexyl, 2-Hexyl, 3-Hexyl, 2-Methyl-1-pentyl, 2-Methyl-2-pentyl, 2-Methyl-3-pentyl, 2-Methyl-4-pentyl, 3-Methyl-1-pentyl, 3-Methyl-2-pentyl oder 3-Methyl-3-pentyl, Allyl, Prenyl, Isoprenyl,
   oder
R² und R³ zusammen einen gesättigten oder ungesättigten carbocyclischen Ring ausbilden, ausgewählt aus der Gruppe bestehend aus: -CH2-CH2-, -CH2-CH2-CH2-, -CH2-CH2-CH2-CH2-, -CH=CH-CH=CH-, -CH2-CH2-CH2-CH2-CH2-, -CH=CH-CH2-CH2-CH2-, -CH2-CH=CH-CH2-CH2-, -CH=CH-CH=CH-CH2-,-CH=CH-CH=CH-CH=.

Eine bevorzugte Ausführungsform sind aromatische Alkensäurederivate der Formel (I) 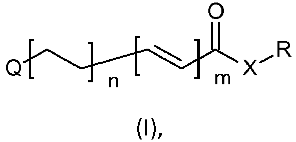 wobei
Q einen Rest 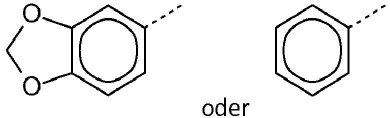 darstellt,
und entweder
i) *n* einen Wert 0 und gleichzeitig m einen Wert 1
   oder
ii) *n* einen Wert 1 und m gleichzeitig einen Wert 1 oder 2 darstellt,
   und
wobei die resultierende(n) Doppelbindung(en) unabhängig voneinander als E-oder Z-Konfiguration vorliegen,
und
X = - OR¹ mit
R¹= Wasserstoff, Methyl, Ethyl, Propyl, 2-Propyl, 1-Butyl, 2-Butyl, 2-Methyl-1-propyl oder - NR²R³ bedeutet,
   und wobei
R² Wasserstoff ist,
   und gleichzeitig
R³ Methyl, Ethyl, 2-Methyl-1-propyl, 2-Methyl-1-butyl, Allyl, Prenyl, Isoprenyl darstellt, oder
R² und R³ zusammen einen gesättigten oder ungesättigten carbocyclischen Ring ausbilden, ausgewählt aus der Gruppe bestehend aus: -CH2-CH2-CH2-CH2-, -CH=CH-CH=CH-, -CH2-CH2-CH2-CH2-CH2-, -CH=CH-CH2-CH2-CH2-, -CH2-CH=CH-CH2-CH2-, -CH=CH-CH=CH-CH2-,-CH=CH-CH=CH-CH=.

Besonders bevorzugt sind aromatische Alkensäurederivate der Formel (I) 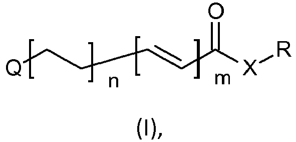 wobei
Q einen Rest 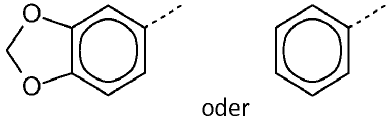 darstellt,
und entweder
i) *n* einen Wert 0 und gleichzeitig *m* einen Wert 1
   oder
ii) *n* einen Wert 1 und *m* gleichzeitig einen Wert 1 oder 2 darstellt,
   und
wobei die resultierende(n) Doppelbindung(en) bevorzugt zu mehr als 50 %, besonders bevorzugt zu mehr als 80 %, ganz besonders bevorzugt zu mehr als 95% in der (*E*)-Konfiguration vorliegen,
und
X = - OR¹ mit
R¹= Wasserstoff, Methyl, Ethyl
oder - NR²R³ bedeutet,
   und wobei
R² Wasserstoff ist
   und gleichzeitig
R³ 2-Methyl-1-propyl oder 2-Methyl-1-butyl ist,
   oder
R² und R³ zusammen einen gesättigten oder ungesättigten carbocyclischen Ring ausbilden, ausgewählt aus der Gruppe bestehend aus: -CH2-CH2-CH2-CH2-, -CH2-CH2-CH2-CH2-CH2-.

Besonders bevorzugt sind hierbei die aromatischen Alkensäurederivate der Formel I ausge-wählt aus der Gruppe bestehend aus:
(2*E*)-*N*-(Cinnamoyl)piperidine (Verbindung 1): 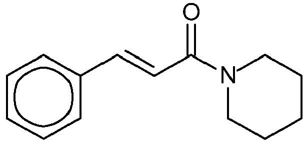
(2*E*)-3-(1,3-Benzodioxol-5-yl)-*N*-isobutyl-prop-2-enamid (*trans*-Fagaramid , Verbindung 2): 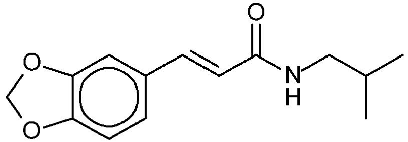
(2*E*)-3-(1,3-Benzodioxol-5-yl)-*N*-piperidinyl-prop-2-enamid (Ilepcimide, Verbindung 3): 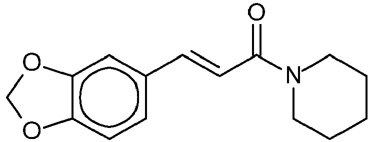
(2*E*)-5-(1,3-Benzodioxol-5-yl)-*N*-isobutyl-pent-2-enamid (Dihydropiperlonguminin, Verbindung 4): 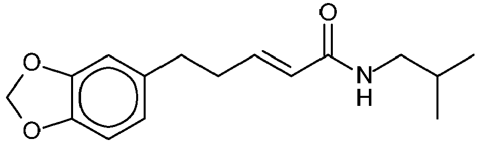
(2*E*,4*E*)-7-(1,3-Benzodioxol-5-yl)-*N*-isobutyl-hepta-2,4-dien-1-amid (Chingchengenamide A, Verbindung 5): 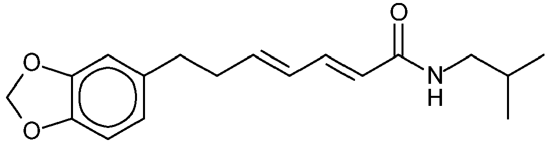
(*2*E,4*E*)-Methyl-7-(1,3-benzodioxol-5-yl)hepta-2,4-dienoat (Verbindung 6):

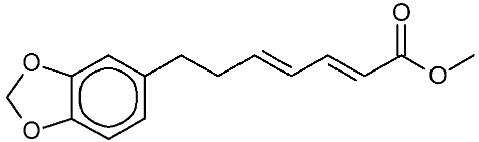

Die erfindungsgemäßen aromatischen Alkensäurederivate (Verbindungen) 1 bis 6 liegen vorzugsweise zu mehr als 50 %, besonders bevorzugt zu mehr als 80 %, ganz besonders bevorzugt zu mehr als 95 % in der E-Konfiguration vor.

Überraschenderweise hat sich gezeigt, dass die erfindungsgemäßen aromatischen Alkensäurdederivate der Formel (I), insbesondere die Verbindungen 1 bis 6 als Geschmackstoffe besonders geeignet sind und einen Beitrag zur Vermittlung, Modifizierung und/oder Verstärkung von Geschmackseindrücken, insbesondere im Bereich Umami, Kokumi und salzig aufweisen. Hierbei können die Verbindungen 1 bis 6 einzeln oder in einer Mischung aus einem, zwei, drei oder mehreren Verbindungen von 1 bis 6, eingesetzt werden.

Besonders in Anbetracht der bekannten brennenden und scharfen Effekte des verwandten Piperins, wie bereits oben beschrieben, war es überraschend, dass die erfindungsgemäßen aromatischen Alkensäurdederivate der Formel (I), speziell die Verbindungen 1 bis 6, trotzdem bei höheren Konzentrationen von 10 bis 100 ppm nur einen unterschwelligen chemesthetischen Reiz vermitteln bzw. erzeugen; dafür jedoch insbesondere in stark Natriumglutamat-reduzierten oder -freien Lebensmitteln, sowie in Lebensmitteln mit reduziertem Natriumchloridgehalt (zum Beispiel in würzigen Lebensmitteln wie Tomatensuppe, Hühnersuppe, Knabbergebäck, Fertigpizza, Kartoffelchips und Popcorn) sowohl im Anfangsgeschmack (Impact) als auch in der länger anhaltenden Geschmackswahrnehmung, insbesondere einen Umami-, Kokumi-artigen oder salzigen Geschmackseindruck vermitteln, modifizieren und/oder verstärken können. Die erfindungsgemäßen aromatischen Alkensäurdederivate der Formel (I), speziell die Verbindungen 1 bis 6, ermöglichen insbesondere die Einstellung eines als besonders angenehm empfundenes Geschmackserlebnisses, welches in vielen Fällen sogar als gegenüber Natriumglutamat bevorzugt bewertet wurde, weil es frei von weiteren unterschwelligen anderen Geschmacksnoten ist.

Demgemäß ist ein weiterer Gegenstand der vorliegenden Erfindung die Verwendung von aromatischen Alkensäurederivate der Formel I, insbesondere der Verbindungen 1 bis 6, einzeln oder als Gemisch aus mehreren Verbindungen, als Geschmackstoffe, insbesondere Geschmackstoffe zur Vermittlung oder Erzielung eines selbstständigen oder Verstärkung eines vorhandenen Umami- oder Kokumi-artigen oder salzigen Geschmackeffekts.

Die erfindungsgemäßen aromatischen Alkensäurederivate sind an sich bekannt und kommen in verschiedenen Gewürzpflanzen vor; dabei ist aber die regelmäßig vorkommende Konzentration in der Regel sehr gering, so dass die bevorzugten erfindungsgemäßen Dosierungen nicht durch einfaches Zumengen des Gewürz oder des einfachen Gewürzextraktes erreicht werden können. Vielmehr müssen die Verbindungen aus diesen Extrakten entsprechend angereichert oder isoliert oder synthetisch gewonnen werden.

Die besonders bevorzugt genannten aromatischen Alkensäurederivate 1 bis 6 kommen alle natürlich, in bestimmten Pflanzenarten vor, wobei einige diese Pflanzen direkt oder in Form von Extrakten und Zubereitungen in Nahrungsmitteln eingesetzt werden können.

Die Pflanzenextrakte enthalten eine oder mehrere der bevorzugt genannten aromatischen Alkensäurederivate und sind ebenfalls Sinne der Erfindung einsetzbar, beispielsweise Pfefferextrakt (*Piper ssp.,* insbesondere *P. nigrum, P. falconeri, P. boehmeriaefolium, P. tuberculatum, P. sintenensis, P. longum, P. chaba* oder *P. guineense),* Extrakte aus Fagara-Arten (*Fagara macrophylla)* sowie Extrakte aus *Macropiper excelsum.*

Die pflanzlichen Extrakte enthaltend eines oder mehrere der aromatischen Alkensäurederivate der Formel I, insbesondere der Verbindungen 1 bis 6. Die aromatischen Alkensäurederivate der Formel I können aus den entsprechenden frischen oder getrockneten Pflanzen oder Pflanzenteilen, insbesondere aber aus weißen, grünen oder schwarzen Pfefferkörnern (*P. nigrum*)*,* Stangenpfeffer (*P. longum*), Pflanzenteilen der Fagara-Arten oder den Blättern, Wurzeln, Ästen, Samen oder Fruchtständen von *Macropiper excelsum* gewonnen werden. Üblicherweise werden die getrockneten Pflanzenteile (z.B. frische oder getrocknete Wurzeln, Früchte, Samen, Rinde, Holz, Stängel, Blätter oder Blüten[teile]), vorzugsweise in zerkleinerter Form, mit einem für Nahrungs- und Genussmittel geeigneten Lösungsmittel bei Temperaturen von 0°C bis zum Siedepunkt des jeweiligen Lösungsmittels oder Lösungsmittelgemisches extrahiert, anschließend filtriert und das Filtrat ganz oder teilweise eingeengt, vorzugsweise durch Destillation, Gefrier- oder Sprühtrocknung. Der so erhaltene Rohextrakt kann dann noch weiter aufgearbeitet werden, beispielsweise enzymatisch behandelt werden, mit Säure (z.B. unter Druck), mit sauren lonentauschern oder mit Wasserdampf behandelt werden, meist bei Drücken von 0,01 mbar bis 100 bar, vorzugsweise bei 1 mbar bis 20 bar, und/oder in einem für Nahrungs- und Genussmittel geeigneten Lösungsmittel aufgenommen werden. Bevorzugt sind Extrakte, in denen die aromatischen Alkensäurederivate den Hauptanteil bezogen auf die Gesamtmenge der Amid-artigen Verbindungen darstellen, beispielsweise 1 bis 90 %, bevorzugt 5 Gew.-%, besonders bevorzugt 10 Gew.-%, besonders bevorzugt 50 Gew.-%, ganz besonders bevorzugt 80 Gew.-% bezogen auf die Gesamtmenge der Amid-artigen Verbindungen darstellt.

Insbesondere für Nahrungs- und Genussmittel zur Extraktion geeignete Lösungsmittel sind Wasser, Ethanol, Methanol, 1,2-Propylenglycol, Glycerin, Aceton, Dichlormethan, Essigsäureethylester, Diethylether, Hexan, Heptan, Triacetin, pflanzliche Öle oder Fette, superkritisches Kohlendioxid und deren Gemische.

Bevorzugte Hilfs- oder Trägerstoffe sind Maltodextrin, Stärke, natürliche oder künstliche Polysaccharide und/oder Pflanzengummen wie modifizierte Stärken oder Gummi Arabi-cum, für die Aromakompositionen zugelassene Lösungsmittel wie z.B. Ethanol, 1,2-Propylenglycol, Wasser, Glycerin, Triacetin, Pflanzenöltriglyceride, färbende Mittel, z.B. zugelassene Lebensmittelfarbstoffe, färbende Pflanzenextrakte, Stabilisatoren, Konservierungsstoffe, Antioxidantien, Viskositäts-beeinflussende Stoffe.

Demgemäß ist ein Pflanzenextrakt, welche Verbindung 6 ((2*E*,4*E*)-Methyl-7-(1,3-benzodioxol-5-yl)hepta-2,4-dienoat) umfasst und Verbindung 6 ((2*E*,4*E*)-Methyl-7-(1,3-benzodioxol-5-yl)hepta-2,4-dienoat) selbst, ebenfalls Gegenstand der vorliegen Erfindung.

Besonders bevorzugt werden die erfindungsgemäßen aromatischen Alkensäurederivate der Formel (I) in Aromamischungen eingesetzt, welche vorzugsweise weitere Aromen oder Geschmackstoffe umfassen. Eine solche Aromamischung stellt im Sinne der vorliegenden Anmeldung eine Halbfertigware dar, da diese nicht das Endprodukt ist, sondern nur ein Zwischenprodukt ist, das vorzugsweise z.B. weiter in oralen Zubereitungen oder Nahrungs- bzw. Lebensmitteln eingearbeitet werden kann.

### Aromamischungen

Demgemäß ist ein weiterer Gegenstand der vorliegenden Erfindung Aromamischungen, umfassend
(a) mindestens ein aromatisches Alkensäurederivat der allgemeinen Formel (I), vorzugsweise mindestens eine Verbindung 1 bis 6, bevorzugt Mischungen aus mindestens zwei, drei oder mehreren der Verbindungen 1 bis 6, und
(b) mindestens einen weiteren Geschmacksstoff.

Der oder die weitere(n) Geschmacksstoff(e) (b) sind selbstverständlich verschieden von der Komponente (a), und sind vorzugsweise so ausgewählt, dass auch die Komponete (b) für sich genommen vorzugsweise mindestens einen der Geschmackseindrücke Umami, Kokumi oder salzig vermitteln und insbesondere in der Lage sind, unangenehme Geschmackseindrücke wie bitter, metallisch, kalkig, sauer oder adstringierend zu maskieren und/oder angenehme Geschmackseindrücke (z.B. süß) zu verstärken.

Geschmacksstoffe, die die Gruppe (b) bilden, sind vorzugsweise ausgewählt aus der Gruppe bestehend aus Mononatriumglutamat, freie Glutaminsäure, Nucleotide (z.B. Adenosin-5'-monophosphat, Cytidin-5'-monophosphat, Inosin-5'-monophosphat, Guanosin-5'-monophosphat) oder deren pharmazeutisch akzeptable Salze, Strombine, Theogalline, Pyridin-Betain-Verbindungen, Glutaminsäureglycoside, Äpfelsäureglycoside, Glutathion-Derivate, Lactisole und Alkylpyridine, insbesondere 2-Hexyl-, 2-Heptyl und 2-Octylpyridin, (2E, 6Z)-N-cyclopropylnona-2,6-dienamid (FEMA 4087; Flavis 16.093), (2E,6Z)-N-ethylnona-2,6-dienamid (FEMA 4113; Flavis 16.094) und N-[(2E)-3,7-dimethylocta-2,6-dienyl]cyclopropancarboxamid (FEMA 4267; Flavis 16.095), N'-[(2-methoxy-4-methylphenyl)methyl]-N-[2-(5-methyl-2-pyridyl)ethyl]oxamid (FEMA 4234; Flavis 16.190), N'-[(2,4-dimethoxyphenyl)methyl]-N-[2-(2-pyridyl)ethyl]oxamid (FEMA 4233; Flavis 16.099), N'-[(2-methoxy-4-methyl-phenyl)methyl]-N-[2-(2-pyridyl)ethyl]oxamid (FEMA 4231; Flavis 16.101), N-(1-propylbutyl)-1,3-benzodioxole-5-carboxamid (FEMA 4232; Flavis 16.098), 1-(2-hydroxy-4-isobutoxy-phenyl)-3-(2-pyridyl)propan-1-on (FEMA 4722) und 1-(2-hydroxy-4-methoxy-phenyl)-3-(2-pyridyl)propan-1-on (FEMA 4723), Zimtsäureamide, insbesondere Rubemamin oder Rubescenamin, Glutathion-Derivate, Hydroxyflavanone (z.B. Eriodictyol, Homoeriodictyol oder deren Natriumsalze), Hesperitin, Phloretin, Hydroxyflavane, 4-Hydroxychalcone, Extrakte auf Basis von Hydrangea dulcis, oder Rubus ssp.; Gemische von Molkeproteinen mit Lecithinen, Hefeextrakte, Pflanzenhydrolysate, pulverisierte Gemüse (z.B. Zwiebelpulver, Tomatenpulver), Pflanzenextrakte (z.B. von Liebstöckel oder von Pilzen wie Shiitake), Meeralgen und Mineralsalzmischungen, insbesondere Mineralsalzmischungen, Rubemamin oder Rubescenamin.

In einer bevorzugten Ausgestaltung der vorliegenden Erfindung umfasst eine erfindungsgemäße Aromamischung
(a) mindestens ein aromatisches Alkensäurederivat der allgemeinen Formel (I), vorzugsweise mindestens eine Verbindung 1 bis 6, bevorzugt Mischungen aus mindestens zwei, drei oder mehreren der Verbindungen 1 bis 6, und
(b) mindestens einen weiteren Geschmacksstoff ausgewählt aus der Gruppe, die gebildet wird von Mononatriumglutamat, freier Glutaminsäure, Nucleotiden oder deren pharmazeutisch akzeptablen Salzen, Strombinen, Theogallinen, Pyridin-Betain-Verbindungen, Glutaminsäureglycosiden, Äpfelsäureglycosiden, Glutathion-Derivaten, Lactisolen und Alkylpyridinen, Hesperetin, 4-Hydroxychalcone, Hydroxyflavanone, 3,7'-Dihydroxy-4'-methoxyflavan, Zimtsäureamiden, Phloretin, Rubemamin, Rubescenamin sowie Gemische daraus.

Hierbei wird bevorzugt ein Verhältnis von Komponente (a) zu (b) in einem Bereich von 10:1 bis 1:10000, bevorzugt von 5:1 bis 1:2000 eingesetzt.

Grundsätzlich können die erfindungsgemäßen Aromamischungen eine oder mehrere aromatische Alkensäurederivate der Formel (I), vorzugsweise mindestens eine Verbindung 1 bis 6, bevorzugt Mischungen aus mindestens zwei, drei oder mehreren der Verbindungen 1 bis 6, in einer Menge von 0,0001 Gew.% bis 95 Gew.%, bevorzugt von 0,1 Gew.% bis 80 Gew.%, besonders bevorzugt von 1 Gew.% bis 50 Gew.-%, bezogen auf das Gesamtgewicht der Aromamischung, umfassen. Die Gesamtmenge der aromatischen Alkensäurederivate der Formel (I) bestimmt sich hierbei durch die Summe aller Verbindungen der Formel (I) in der Aromamischung.

Die Einarbeitung der erfindungsgemäßen Aromamischungen in oralen Zubereitungen, worunter auch Nahrungs- und Lebensmitteln sowie Halbfertigwaren im Sinne der vorliegende Erfindung, zu verstehen ist, ist besonders vorteilhaft, da die erfindungsgemäßen Aromamischungen das Geschmacksprofil des Produktes (Nahrungs- und Lebensmittel sowie Halbfertigware), insbesondere im Bereich würzig und/oder salzig verstärkt, vertieft und abrundet.

Besonders bevorzugt sind Aromamischungen, bei denen der mindestens weiterer Geschmackstoff ausgewählt ist aus Mononatriumglutamat, freier Glutaminsäure, Nucleotiden oder deren pharmazeutisch akzeptablen Salzen, Rubemamin und/oder Rubescenamin, Hesperetin, 3,7'-Dihydroxy-4'-methoxyflavan und/oder Phloretin. Die Aromamischungen werden in diesen Fällen vorzugsweise so eingesetzt, dass der Gesamtanteil an Mononatriumglutamat, freier Glutaminsäure, Nucleotiden oder deren pharmazeutisch akzeptablen Salzen im Bereich von 10 ppm bis 10000 ppm, bevorzugt im Bereich von 20 ppm bis 5000 ppm in der Endzubereitung (Nahrungs- und Lebensmittel bzw. Halbfertigware und/oder Rubemamin und/oder Rubescenamin, Hesperetin, 3,7'-Dihydroxy-4'-methoxyflavan und/oder Phloretin im Bereich von 1 ppm bis 400 ppm, bevorzugt im Bereich von 5 ppm bis 200 in der Endzubereitung (Nahrungs- und Lebensmittel) bzw. Halbfertigware vorliegt, bezogen auf das Gesamtgewicht der (Nahrungs- und Lebensmittel) Zubereitung bzw. Halbfertigware.

### Orale Zubereitungen

Ein weiterer Gegenstand der vorliegenden Erfindung umfasst orale Zubereitungen, umfassend
(a) mindestens eine erfindungsgemäße (wie oben beschriebene) Aromamischung
   oder
(b) mindestens ein aromatisches Alkensäurederivat der Formel (I), vorzugsweise mindestens eine Verbindung 1 bis 6, bevorzugt Mischungen aus mindestens zwei, drei oder mehreren der Verbindungen 1 bis 6,
   und
(c) mindestens einen weiteren zum Verzehr geeigneten Bestandteil ausgewählt aus der Gruppe die gebildet wird Trägerstoffen, Aromastoffen, Fetten und Ölen, Emulgatoren und Antioxidantien, weitere Hilfs- und Zusatzstoffe, Natriumglutamat, Zucker und Salz.

Die erfindungsgemäßen oralen Zubereitungen stellen gebrauchs- oder verzehrfertige (der Ernährung oder dem Genuss dienenden) Zubereitungen dar (Nahrungs- und Lebensmittel). Dabei handelt es sich um Produkte, die dazu bestimmt sind, in die menschliche Mundhöhle eingebracht zu werden, dort eine bestimmte Zeit zu verbleiben und anschließend entweder verzehrt (z.B. verzehrfertige Lebensmittel, siehe auch weiter unten) oder wieder aus der Mundhöhle entfernt zu werden (z.B. Kaugummis). Zu diesen Produkten gehören dabei sämtliche Stoffe oder Erzeugnisse, die dazu bestimmt sind, in verarbeitetem, teilweise verarbeitetem oder unverarbeitetem Zustand vom Menschen aufgenommen zu werden. Hierzu zählen auch Stoffe, die Lebensmitteln bei ihrer Herstellung, Verarbeitung oder Bearbeitung zugesetzt werden und dazu vorgesehen sind, in die menschliche Mundhöhle eingebracht zu werden.

Bevorzugt sind hierbei orale Zubereitungen, umfassend
(a) mindestens eine erfindungsgemäße (wie oben beschriebene) Aromamischung
   oder
(b) mindestens ein aromatisches Alkensäurederivat der Formel (I), vorzugsweise mindestens eine Verbindung 1 bis 6, bevorzugt Mischungen aus mindestens zwei, drei oder mehreren der Verbindungen 1 bis 6,
   und
(c) mindestens einen weiteren zum Verzehr geeigneten Bestandteil ausgewählt aus der Gruppe die gebildet wird Trägerstoffen, Aromastoffen, Fetten und Ölen, Emulgatoren und Antioxidantien, weitere Hilfs- und Zusatzstoffe, Natriumglutamat, Zucker und Salz,
wobei die Menge der aromatischen Alkensäurederivate der Formel (I) in einem Bereich von von 5 bis 500 ppm, bevorzugt von 10 bis 250 ppm, besonders bevorzugt von 10 ppm bis 100 ppm in der Zubereitung vorliegt, bezogen auf das Gesamtgewicht der oralen Zubereitungen, wobei sich die Menge der aromatischen Alkensäurederivate der Formel (I) aus der Summe aller aromatischen Alkensäurderivate der Formel (I) in der Zubereitung ergibt.

### Halbfertigwaren

Der Begriff der oralen Zubereitung umfasst im Sinne der Erfindung auch Halbfertigwaren zur Herstellung einer der Ernährung oder dem Genuss dienenden Zubereitung Unter einer Halbfertigware ist im Zusammenhang mit der vorliegenden Erfindung ein Produkt zu verstehen, welches aufgrund eines sehr hohen Gehaltes an Aroma- und Geschmacksstoffen für die Verwendung als verzehrfertiges Lebensmittel ungeeignet ist. Erst durch Mischen mit mindestens einem weiteren Bestandteil (d.h. durch Verringern der Konzentration der betreffenden Aroma- und Geschmacksstoffe) und gegebenenfalls weitere Prozessschritte (z.B. Erwärmen, Gefrieren) wird die Halbfertigware in ein verzehrfertiges Lebensmittel überführt. Als Beispiel für Halbfertigwaren seien hier Tütensuppen, Backaromen und Puddingpulver genannt.

Halbfertigwaren, umfassen vorzugsweise aromatische Alkensäurederivate der Formel (I) in einer Menge im Bereich von 50 ppm bis 500.000 ppm, bevorzugt 100 ppm bis 250.000 ppm, besonders bevorzugt 1000 ppm bis 10.000 ppm in der Halbfertigware, bezogen auf das Gesamtgewicht der Halbfertigware.

Ebenfalls möglich ist die Verwendung von Aromamischungen gemäß der vorliegenden Erfindung in Halbfertigwaren. Werden erfindungsgemäße Aromamischungen eingesetzt, so werden diese ebenfalls so verwendet, dass die Menge der aromatischen Alkensäurederivate der Formel (I) in einem Bereich von 50 ppm bis 500.000 ppm, bevorzugt von 100 ppm bis 250.000 ppm, besonders bevorzugt von 1000 ppm bis 10.000 ppm in der Halbfertigware vorliegt, bezogen auf das Gesamtgewicht der Halbfertigware.

Halbfertigwaren werden vorzugsweise in einer Konzentration von 0,01 Gew.% bis 10 Gew.-%, bevorzugt von 0,05 Gew.% bis 5 Gew.-%, besonders bevorzugt von 0,05 Gew.% bis 1 Gew.-%, bezogen auf das verzehrsfertige Lebensmittel (Endprodukt), eingesetzt.

### Nahrungs- oder Lebensmittel

Im Rahmen der vorliegenden Erfindung wird unter einem "Nahrungs- oder Lebensmittel" insbesondere Stoffe verstanden, die dazu bestimmt sind, in unverändertem, zubereitetem oder verarbeitetem Zustand vom Menschen geschluckt und dann verdaut zu werden; als Lebensmittel werden insoweit auch Umhüllungen, Überzüge oder sonstige Umschließungen verstanden, die dazu bestimmt sind, mitverschluckt zu werden, oder bei denen ein Verschlucken vorauszusehen ist. Auch bestimmte Produkte, die üblicherweise wieder aus der Mundhöhle entfernt werden (z.B. Kaugummis) sind im Rahmen des vorliegenden Textes als Lebensmittel zu verstehen, da bei ihnen nicht auszuschließen ist, dass sie zumindest teilweise geschluckt werden.

Unter einem "verzehrfertigen Lebensmittel" ist hierbei ein Lebensmittel zu verstehen, das hinsichtlich der für den Geschmack maßgeblichen Substanzen bereits vollständig zusammengesetzt ist. Unter den Begriff "verzehrfertiges Lebensmittel" fallen auch Getränke sowie feste oder halbfeste verzehrfertige Lebensmittel. Als Beispiele seien genannt Tiefkühlprodukte, die vor dem Verzehr aufgetaut und auf Verzehrtemperatur erwärmt werden müssen. Auch Produkte wie Joghurt oder Eiscreme aber auch Kaugummis oder Hartkaramellen zählen zu den verzehrfertigen Lebensmitteln.

Bevorzugt werden die erfindungsgemäßen aromatischen Alkensäurederivate der allgemeinen Formel (I) oder die erfindungsgemäßen Aromamischungen mit einem oder mehreren aromatischen Alkensäurederivate der Formel (I), in einer Menge von 5 ppm bis 500 ppm, bevorzugt von 10 ppm bis 250 ppm, besonders bevorzugt von 10 ppm bis 100 ppm in Nahrungs- bzw. Lebensmitteln eingesetzt, bezogen auf das Gesamtgewicht der Nahrungs- oder Lebensmitteln, wobei sich die Menge der aromatischen Alkensäurederivate der Formel (I) aus der Summe aller aromatischen Alkensäurderivate der Formel (I) im Nahrungs- bzw. Lebensmittel, ergibt.

### A. Trägerstoffe

Eine Reihe von erfindungsgemäßen Zubereitungen oder Halbfertigwaren (wie oben beschrieben) ist besonders bevorzugt. So sind beispielsweise erfindungsgemäße Zubereitungen oder Halbfertigwaren gemäß einer bevorzugten Ausführungsform sprühgetrocknete Zubereitungen bzw. Halbfertigwaren, die als weitere zum Verzehr geeignete Bestandteile (unter anderem) feste Trägerstoffe umfassen.

Erfindungsgemäß bevorzugt sind erfindungsgemäße Zubereitungen oder Halbfertigwaren, die einen oder mehrere zum Verzehr geeignete feste Trägerstoffe umfassen, wobei, bezogen auf die Trockenmasse der Zubereitung bzw. Halbfertigware, das Gewichtsverhältnis der Gesamtmenge an aromatischen Alkensäurederivaten der Formel (I) zu der Gesamtmenge an zum Verzehr geeigneten festen Trägerstoffen vorzugsweise im Bereich von 1 : 1 bis 1 : 100000, bevorzugt im Bereich von 1 : 5 (vorzugsweise 1 : 100) bis 1 : 20000, besonders bevorzugt im Bereich von 1 : 10 (vorzugsweise 1 : 1000) bis 1 : 5000, liegt.

Weiter bevorzugt ist es, wenn dabei die Gesamtmenge von aromatischen Alkensäurederivaten der Formel (I) und zum Verzehr geeigneten festen Trägerstoffen bezogen auf das Gesamtgewicht der Zubereitung bzw. Halbfertigware im Bereich von 70 bis 100 Gew.-%, bevorzugt im Bereich von 85 bis 100 Gew.-%, liegt.

Vorteilhafte Trägerstoffe sind Siliciumdioxid (Kieselsäure, Kieselgel), Kohlenhydrate und/oder Kohlenhydratpolymere (Polysaccharide), Cyclodextrine, Stärken, abgebaute Stärken (Stärkehydrolysate), chemisch oder physikalisch modifizierte Stärken, modifizierte Cellulosen, Gummi Arabicum, Ghatti-Gummi, Traganth, Karaya, Carrageenan, Guarkernmehl, Johannisbrotkernmehl, Alginate, Pektin, Inulin oder Xanthan Gum. Bevorzugte Stärkehydrolysate sind Maltodextrine und Dextrine.

Erfindungsgemäß besonders bevorzugte Trägerstoffe sind Siliciumdioxid, Gummi Arabicum und Maltodextrine, wobei hier wiederum Maltodextrine mit DE-Werten im Bereich 5 bis 20 besonders bevorzugt sind. Es ist dabei unerheblich, welche Pflanze ursprünglich die Stärke zur Herstellung der Stärkehydrolysate geliefert hat. Besonders geeignet und leicht verfügbar sind jedoch Mais-basierende Stärken sowie Stärken aus Tapioka, Reis, Weizen oder Kartoffeln. Die Trägerstoffe können dabei auch als Fließhilfsmittel fungieren, wie beispielsweise Siliciumdioxid.

Die erfindungsgemäßen Zubereitungen oder Halbfertigwaren, die neben der oder den erfindungsgemäßen aromatischen Alkensäurederivate der Formel (I) bzw. oben definierten Mischungen noch einen oder mehrere feste Trägerstoffe umfassen, können beispielsweise durch mechanische Mischvorgänge, wobei gleichzeitig auch eine Zerkleinerung der Partikel erfolgen kann, oder mittels Sprühtrocknung hergestellt werden. Besonders bevorzugt sind dementsprechend erfindungsgemäße Zusammensetzungen oder Halbfertigwaren, die feste Trägerstoffe umfassen und mittels Sprühtrocknung hergestellt sind; welche beispielsweise aus US 3,159,585, US 3,971,852, US 4,532,145 oder US 5,124,162 bekannt sind.

Bevorzugte erfindungsgemäße, Trägerstoffe umfassende Zubereitungen und Halbfertigwaren, die mittels Sprühtrocknung hergestellt werden, besitzen eine mittlere Partikelgröße im Bereich von 30 µm bis 300 µm und eine Restfeuchte von kleiner oder gleich 5 Gew.-%.

### B. Aromastoffe

Die oralen Zubereitungen in der vorliegenden Erfindung können als weitere Bestandteile einen oder mehrere weitere Aromastoffe umfassen. Eine solche Aromakomposition aus mehreren Aromastoffen, umfasst für die Zwecke der vorliegenden Erfindung mindestens einen flüchtigen Aromastoff (aromatischen Alkensäurederivate der Formel (I) sind hier natürlich ausgenommen). In Zubereitungen im Nahrungs- und Lebensmittelbereich werden Aroma (-stoffe) und Geschmack als Gesamtaroma wahrgenommen, so dass keine eindeutige Trennung zwischen Aromastoffe und Geschmacksstoffe vorliegt. Demgemäß sind diese beiden Stoffgruppen, für die vorliegende Erfindung in eine Stoffgruppe (= Aromastoffe) zusammenzufassen.

Demgemäß umfassen geeignete Aromakompositionen für die Verwendung in erfindungsgemäßen Zubereitungen, Halbfertigwaren oder Aromamischungen vorzugsweise synthetische, natürliche oder naturidentische Aroma-, Riech- und Geschmacksstoffe, Reaktionsaromen, Raucharomen oder andere aromagebende Zubereitungen (z.B. Protein[teil]hydrolysate, Grillaromen, Pflanzenextrakte, Gewürze, Gewürzzubereitungen, Gemüse und/oder Gemüsezubereitungen) sowie gegebenenfalls geeignete Hilfs- und Trägerstoffe. Insbesondere sind hier solche Aromenkompositionen oder deren Bestandteile geeignet, die einen röstigen, fleischigen (insbesondere Huhn, Fisch, Meerestiere, Rind, Schwein, Lamm, Schaf, Ziege), gemüsigen (insbesondere Tomate, Zwiebel, Knoblauch, Sellerie, Lauch, Pilze, Auberginen, Seetang), einen würzigen (insbesondere schwarzer und weißer Pfeffer, Chili, Paprika, Kardamom, Muskat, Piment, Senf und Senf-Produkte), gebratenen, hefigen, gesottenen, fettigen, salzigen und/oder scharfen Aromaeindruck verursachen und somit den würzigen Eindruck verstärken können. In der Regel enthalten die Aromakompositionen mehr als einen der genannten Inhaltsstoffe.

Modulierende Aroma- und/oder Geschmackstoffe werden vorzugsweise ausgewählt aus der Gruppe bestehend aus Adenosin-5'-monophosphat, Cytidin-5'-monophosphat, Inosin-5'-monophosphat, und deren pharmazeutisch akzeptablen Salzen; Lactisolen; 2,4-Dihydroxybenzoesäure; 3-Hydroxybenzoesäure; Natriumsalzen, vorzugsweise Natriumchlorid, Natriumlactat, Natriumcitrat, Natriumacetat, Natriumgluconoat; Hydroxyflavanonen, wie zum Beispiel Eriodictyol, Homoeriodictyol, und deren Natriumsalzen; Hydroxybenzoesäureamiden, wie zum Beispiel 2,4-Dihydroxybenzoesäurevanillylamid, 2,4-Dihydroxybenzoesäure-N-(4-hydroxy-3-methoxybenzyl)amid, 2,4,6-Trihydroxybenzoesäure-*N-*(4-hydroxy-3-methoxybenzyl)amid, 2-Hydroxy-benzoesäure-*N*-4-(hydroxy-3-methoxybenzyl)amid, 4-Hydroxybenzoesäure-*N*-(4-hydroxy-3-methoxybenzyl)-amid, 2,4-Dihydroxybenzoesäure-*N*-(4-hydroxy-3-methoxybenzyl)amid-Mono-natriumsalz, 2,4-Dihydroxybenzoesäure-N-2-(4-hydroxy-3-methoxyphenyl)-ethyl-amid, 2,4-Dihydroxybenzoesäure-*N-*(4-hydroxy-3-ethoxybenzyl)amid, 2,4-Dihydroxybenzoesäure-*N*-(3,4-dihydroxybenzyl)amid und 2-Hydroxy-5-methoxy-N-[2-(4-hydroxy-3-methoxyphenyl)ethyl]amid; 4-Hydroxybenzoesäurevanillylamid; Hydroxydeoxybenzoinen, wie zum Beispiel 2-(4-Hydroxy-3-methoxyphenyl)-1-(2,4,6-trihydroxyphenyl)ethanon, 1-(2,4-Dihydroxyphenyl)-2-(4-hydroxy-3-methoxyphenyl)ethanon, 1-(2-Hydroxy-4-methoxyphenyl)-2-(4-hydroxy-3-methoxyphenyl) ethanon); Hydroxyphenlaalkandionen, wie zum Beispiel Gingerdion-[2], Gingerdion-[3], Gingerdion-[4], Dehydrogingerdion-[2], Dehydrogingerdion-[3], Dehydrogingerdion-[4]); Diacetyltrimeren; γ-Aminobuttersäuren und Divanillinen; Bicyclo[4.1.0]heptan-7-carbonsäureamide; Cyclopropancarbonsäure(3-methyl-cyclohexyl)amide, aromatische *Neo-*Menthylamide; Geranylaminederivate der Oxalsäure sowie Neomenthylderivate.

Die Aromakompositionen können auch in Form von Reaktionsaromen (Maillard-Produkte) und/oder Extrakten bzw. ätherischen Ölen von Pflanzen oder Pflanzenteilen bzw. Fraktionen davon eingesetzt werden.

### C. Fette und Öle

Gemäß einer weiteren bevorzugten Ausgestaltung der vorliegenden Erfindung können die oralen Zubereitungen Fette und Öle enthalten und beispielsweise als Wasser-in-ÖI (W/O)-Emulsionen vorliegen.

Neben der oder den erfindungsgemäßen aromatischen Alkensäurederivaten der Formel (I), oder die daraus erhältlichen Aromazubereitungen, umfasst eine solche Emulsion Wasser, eine Ölphase, ein oder mehrere (verzehrbare) W/O-Emulgatoren, gegebenenfalls ein oder mehrere Antioxidantien und gegebenenfalls ein oder mehrere Stoffe zur Verstärkung einer antioxidativen Wirkung. Die Ölphase einer solchen erfindungsgemäßen W/O-Emulsion besteht aus oder umfasst vorzugsweise ein fettes Öl und/oder eine Aromakomposition (vorzugsweise eine wie oben beschriebene Aromakomposition).

Als fette Öle eignen sich beispielsweise Speiseöle, insbesondere Pflanzenöle. Geeignete fette Öle sind beispielsweise Borretschöl, Distelöl, Erdnussöl, Haselnussöl, Kokosöl, Kürbiskernöl, Leinöl, Maiskeimöl, Makadamianussöl, Mandelöl, Olivenöl, Palmkernöl, Pekannussöl, Pistazienkernöl, Rapsöl, Reiskeimöl, Sesamöl, Sojaöl, Sonnenblumenöl, Walnussöl oder Weizenkeimöl, oder daraus erhältliche Fraktionen. Es können auch flüssige Neutralester basierend auf mittelkettigen Fettsäuren und Glycerin verwendet werden, wie beispielsweise Miglyole (z.B. Miglyol 810, Miglyol 812). Bevorzugt sind Sonnenblumenöl, Palmkernöl und Rapsöl. Ferner bevorzugt werden fraktionierte Kokosöle verwendet, die hauptsächlich Fettsäurereste mit 6 bis 8 C-Atomen aufweisen. Diese zeichnen sich durch ihre Geschmacksneutralität sowie durch ihre gute Oxidationsstabilität aus.

Die erfindungsgemäßen W/O-Emulsionen eignen sich besonders zum Aufbringen auf Lebensmitteloberflächen, wobei die Lebensmittel vorzugsweise einen Wassergehalt von höchstens 10 Gew.-%, bevorzugt von höchstens 5 Gew.-% aufweisen. In einer bevorzugten Ausführungsform weist die erfindungsgemäße W/O-Emulsion bei Aufbringungstemperatur eine ausreichend niedrige Viskosität auf, so dass ein Aufbringen der W/O-Emulsion mittels Sprühen möglich ist. Bevorzugte Lebensmittel, auf deren Oberflächen eine erfindungsgemäße W/O-Emulsion aufgebracht werden kann sind beispielsweise Cracker, Chips (z.B. auf Basis von Kartoffeln, Mais, Getreide oder Brot), extrudierte Knabberartikel (Snacks, z.B. Flips) oder Laugengebäck (z.B. Salzstangen). Erfindungsgemäße W/O-Emulsionen werden regelmäßig in einer Menge von 0,5Gew.% bis 6 Gew.-% auf die Lebensmitteloberflächen aufgebracht, bezogen auf das Gesamtgewicht des Lebensmittels.

### D. W/O-Emulgatoren

Zur Stabilisierung der Fett- bzw. Öl/Wassergemische werden für Lebensmittel zugelassene W/O-Emulgatoren benötigt. Vorzugsweise wird der verzehrbare W/O-Emulgator gewählt aus der Gruppe bestehend aus Lecithin (E 322), Mono- und Diglyceride von Speisefettsäuren (E 471), Essigsäuremonoglyceride (E 472a), Milchsäuremonoglyceride (E 472b), Citronensäuremonoglyceride (E 472c), Weinsäuremonoglyceride (E 472d), Diacetylweinsäuremonoglyceride (E 472e), Sorbitanmonostearat (E 491).

### E. Antioxidantien

Die Zubereitungen können des Weiteren auch Antioxidantien enthalten. Geeignete Antioxidantien und Stoffe, welche die antioxidative Wirkung verstärken können sind insbesondere die natürlich vorkommenden Tocopherole und deren Derivate, Tocotrienole, Flavonoide, Ascorbinsäure und ihre Salze, alpha-Hydroxysäuren (z.B. Citronensäure, Milchsäure, Äpfelsäure, Weinsäure) und deren Na-, K- und Ca-Salze, aus Pflanzen isolierte Inhaltsstoffe, Extrakte bzw. Fraktionen davon, z.B. aus Tee, Grüntee, Algen, Traubenkernen, Weizenkeimen, Rosmarin, Oregano, Flavonoide, Quercetin, phenolische Benzylamine. Weiterhin sind als Antioxidantien Propylgallat, Octylgallat, Dodecylgallat, Butylhydroxyanisol (BHA), Butylhydroxytoluol (BHT), Lecithine, Mono- und Diglyceride von Speisefettsäuren verestert mit Citronensäure, Orthophosphate und Na-, K- und Ca-Salze der Monophosphorsäure und Ascorbylpalmitat geeignet.

### F. Weitere Hilfs- und Zusatzstoffe

Weitere Bestandteile einer erfindungsgemäßen, der Ernährung oder dem Genuss dienenden verzehrfertigen Zubereitung oder einer erfindungsgemäßen Halbfertigware können übliche Grund-, Hilfs- und Zusatzstoffe für Nahrungs- oder Genussmittel sein, z.B. Wasser, Gemische frischer oder prozessierter, pflanzlicher oder tierischer Grund- oder Rohstoffe (z.B. rohes, gebratenes, getrocknetes, fermentiertes, geräuchertes und/oder gekochtes Fleisch, Knochen, Knorpel, Fisch, Gemüse, Kräuter, Nüsse, Gemüsesäfte oder -pasten oder deren Gemische), verdauliche oder nicht verdauliche Kohlenhydrate (z.B. Saccharose, Maltose, Fructose, Glucose, Dextrine, Amylose, Amylopektin, Inulin, Xylane, Cellulose, Tagatose), Zuckeralkohole (z.B. Sorbit, Erythritol), natürliche oder gehärtete Fette (z.B. Talg, Schmalz, Palmfett, Kokosfett, gehärtetes Pflanzenfett), Öle (z.B. Sonnenblumenöl, Erdnussöl, Maiskeimöl, Olivenöl, Fischöl, Sojaöl, Sesamöl), Fettsäuren oder deren Salze (z.B. Kaliumstearat), proteino-gene oder nicht-proteinogene Aminosäuren und verwandte Verbindungen (z.B. γ-Aminobuttersäure, Taurin), Peptide (z.B. Glutathion), native oder prozessierte Proteine (z.B. Gelatine), Enzyme (z.B. Peptidasen), Nucleinsäuren, Nucleotide, Geschmackskorrigenzien für unangenehme Geschmackseindrücke, weitere Geschmacksmodulatoren für weitere, in der Regel nicht unangenehme Geschmackseindrücke, andere geschmacksmodulierende Stoffe (s. hierzu auch oben; z.B. Inositolphosphat, Nucleotide wie Guanosinmonophosphat, Adenosinmonophosphat oder andere Stoffe wie Natriumglutamat oder 2-Phenoxypropionsäure), Emulgatoren (z.B. Lecithine, Diacylglycerole, Gummi arabicum), Stabilisatoren (z.B. Carrageenan, Alginat), Konservierungsstoffe (z.B. Benzoesäure und deren Salze, Sorbinsäure und deren Salze), Antioxidantien (z.B. Tocopherol, Ascorbinsäure), Chelatoren (z.B. Citronensäure), organische oder anorganische Säuerungsmittel (z.B. Essigsäure, Phosphorsäure), zusätzliche Bitterstoffe (z.B. Chinin, Coffein, Limonin, Amarogentin, Humolone, Lupolone, Catechine, Tannine), die enzymatische Bräunung verhindernde Stoffe (z.B. Sulfit, Ascorbinsäure), etherische Öle, Pflanzenextrakte, natürliche oder synthetische Farbstoffe oder Farbpigmente (z.B. Carotinoide, Flavonoide, Anthocyane, Chlorophyll und deren Derivate), Gewürze, trigeminal wirksame Stoffe oder Pflanzenextrakte, enthaltend solche trigeminal wirksamen Stoffe, synthetische, natürliche oder naturidentische Aromastoffe oder Riechstoffe sowie Geruchskorrigenzien.

Bevorzugt enthalten erfindungsgemäße Zubereitungen oder Halbfertigwaren zudem eine Aromakomposition (wie oben beschrieben), um den Geschmack und/oder den Geruch abzurunden und zu verfeinern.

### G. Natriumglutamat, Zucker und Salz

Besonders bevorzugt werden die erfindungsgemäßen aromatischen Alkensäurederivate der Formel (I), sowie die daraus erhältlichen Aromazubereitungen in Lebens- und Nahrungsmitteln oder Halbfertigwaren eingesetzt, die zum einen
(a) entweder einen reduzierten Gehalt an Natriumglutamat aufweisen oder völlig frei davon sind,
   und / oder
(b) entweder einen reduzierten Gehalt an Salz aufweisen oder ebenfalls völlig frei davon sind.

Insbesondere bevorzugt sind Lebens- und Nahrungsmitteln, insbesondere orale Zubereitungen oder Halbfertigwaren, die sowohl Natriumglutamat-reduziert oder Natriumglutamat - frei und Salz-reduziert oder Salz-frei sind.

Der Begriff "Natriumglutamat-reduziert" bedeutet in der vorliegenden Erfindung, dass die erfindungsgemäßen Zubereitungen oder Halbfertigwaren deutlich weniger Natriumglutamat enthält, als die Zubereitungen bzw. Halbfertigwaren üblicherweise enthalten, um denselben Geschmackseffekt zu erzielen. In einer solchen "Natriumglutamat-reduziert" Natriumglutamatgehalt liegt dabei um 5 bis weniger als 100 Gew.-%, bevorzugt 10 bis 50 Gew.-%, besonders bevorzugt 20 bis 50 Gew.-% unter dem Natriumglutamatgehalt der üblichen Zubereitung bzw. Halbfertigware. Sofern neben einer oder mehreren aromatischen Alkensäurederivaten der Formel (I) auch Natriumglutamat in einer erfindungsgemäßen Zubereitung oder Halbfertigware vorliegt, liegt das Gewichtsverhältnis der Gesamtmenge an aromatischen Alkensäurederivate der Formel (I) zu Natriumglutamat vorzugsweise im Bereich von 1:1 bis 1:200. Diese Definition gilt mutatis-mutandis auch für Salz-reduzierte Zubereitungen.

Besonders bevorzugt sind demnach solche Natriumglutamat-reduzierten erfindungsgemäßen Zubereitungen oder Halbfertigwaren, wobei das Gewichtsverhältnis der Gesamtmenge an aromatischen Alkensäurederivaten der Formel (I) zur Gesamtmenge an Natriumglutamat, bezogen auf die Trockenmasse der Zubereitung bzw. Halbfertigware, im Bereich von 1 : 1 bis 1 : 200 liegt. Auch hier gilt die bevorzugte Ausführungsform analog für Salz-reduzierten Zubereitungen.

Besonders bevorzugt sind solche Natriumglutamat-reduzierte Zubereitungen, bei denen
- die Gesamtmenge an Natriumglutamat nicht ausreicht, um in einer Vergleichszubereitung, die keine aromatischen Alkensäurederivate der Formel (I) umfasst, aber ansonsten identisch zusammengesetzt ist (normale Natriumglutamat-reduzierte Zubereitung), einen (befriedigenden) Umami-Geschmackseindruck zu vermitteln,
- und die Gesamtmenge an aromatischen Alkensäurederivaten der Formel (I) ausreicht, um der Zubereitung einen (befriedigenden) Umami-Geschmackseindruck sowie optional einen (befriedigenden) Kokumi- und/oder einen (befriedigenden) salzigen Geschmackseindruck zu vermitteln.

Der Ernährung oder dem Genuss dienende erfindungsgemäße Zubereitungen und erfindungsgemäße Halbfertigwaren sind vorzugsweise ausgewählt aus der Gruppe bestehend aus
- Backwaren (z.B. Brot, Trockenkekse, Kuchen, sonstiges Gebäck),
- Getränken (z.B. Gemüsesäfte, Gemüsesaftzubereitungen),
- Instantgetränken (z.B. Instant-Gemüsegetränke),
- Fleischprodukten (z.B. Schinken, Frischwurst- oder Rohwurstzubereitungen, gewürzte oder marinierte Frisch- oder Pökelfleischprodukte),
- gewürzten oder marinierten Fischprodukten (z.B. Surimi),
- Eier oder Eiprodukten (Trockenei, Eiweiß, Eigelb),
- Getreideprodukten (z.B. vorgegarte Fertigreis-Produkte, Reismehlprodukte, Hirse- und Sorghum-Produkte, rohe oder vorgegarte Nudeln und Pastaprodukte),
- Milchprodukten (z.B. Frischkäse, Weichkäse, Hartkäse, Milchgetränke, Molke, Butter, teilweise oder ganz hydrolysierte Milchprotein-haltige Produkte),
- Produkten aus Sojaprotein oder anderen Sojabohnen-Fraktionen (z.B. Sojamilch und daraus gefertigte Produkte, Sojalecithin-haltige Zubereitungen, fermentierte Produkte wie Tofu oder Tempe oder daraus gefertigte Produkte, Sojasoßen),
- Gemüsezubereitungen (z.B. Ketchup, Soßen, Trockengemüse, Tiefkühlgemüse, vorgegarte Gemüse, in Essig eingelegte Gemüse, Gemüsekonzentrate oder -pasten, eingekochte Gemüse, Kartoffelzubereitungen),
- Knabberartikeln (z.B. gebackene oder frittierte Kartoffelchips oder Kartoffelteigprodukte, Brotteigprodukte, Extrudate auf Mais-, Reis- oder Erdnussbasis),
- Produkten auf Fett- und Ölbasis oder Emulsionen derselben (z.B. Mayonnaise, Aufstrich, Remoulade, Dressings, Würzzubereitungen),
- sonstigen Fertiggerichten und Suppen (z.B. Trockensuppen, Instant-Suppen, vorgegarte Suppen), Soßen (Instant-Soßen, Trockensoßen, Fertigsoßen),
- Gewürzen oder Gewürzzubereitungen (z.B. Senfzubereitungen, Meerrettichzubereitungen), Würzmischungen sowie insbesondere
- Aufstreuwürzungen (englisch: Seasonings), die beispielsweise im Snackbereich Anwendung finden.

Natriumglutamat-reduzierte oder -freie der Ernährung oder dem Genuss dienende erfindungsgemäße Zubereitungen und Halbfertigwaren sind vorzugsweise ausgewählt aus der Gruppe bestehend aus Backwaren (z.B. Brot, Trockenkekse, Kuchen, sonstiges Gebäck), Gemüsesaftzubereitungen, Fleischprodukte (z.B. Schinken, Frischwurst- oder Rohwurstzubereitungen, gewürzte oder marinierte Frisch- oder Pökelfleischprodukte), gewürzte oder marinierte Fischprodukte (z.B. Surimi), Eier oder Eiprodukte (Trockenei, Eiweiß, Eigelb), Getreideprodukte (z.B. vorgegarte Fertigreis-Produkte, rohe oder vorgegarte Nudeln und Pastaprodukte), Milchprodukte (z.B. Frischkäse, Weichkäse, Hartkäse, Milchgetränke, Molke, Butter, teilweise oder ganz hydrolysierte Milchprotein-haltige Produkte), Produkte aus Sojaprotein oder anderen Sojabohnen-Fraktionen (z.B. Sojamilch und daraus gefertigte Produkte, Sojalecithin-haltige Zubereitungen, fermentierte Produkte wie Tofu oder Tempe oder daraus gefertigte Produkte, Sojasoßen), Fischsoßen wie z.B. Anchovis-Soßen, Austernsoßen, Gemüsezubereitungen (z.B. Ketchup, Soßen, Trockengemüse, Tiefkühlgemüse, vorgegarte Gemüse, in Essig eingelegte Gemüse, eingekochte Gemüse, Kartoffelzubereitungen), Knabberartikel (z.B. gebackene oder frittierte Kartoffelchips oder Kartoffelteigprodukte, Brotteigprodukte, Extrudate auf Mais- oder Erdnussbasis), Produkte auf Fett- und Ölbasis oder Emulsionen derselben (z.B. Mayonnaise, Aufstrich, Remoulade, Dressings, Würzzubereitungen), Fertiggerichte, Suppen (z.B. Trockensuppen, Instant-Suppen, vorgegarte Suppen), Brühwürfel, Soßen (Instant-Soßen, Trockensoßen, Fertigsoßen), Gewürze, Würze, Würzmittel, Würzmischungen sowie insbesondere Aufstreuwürzungen (englisch: Seasonings), die beispielsweise im Snackbereich Anwendung finden.

Die erfindungsgemäßen Zubereitungen und Halbfertigwaren können auch in Form von Kapseln, Tabletten (nichtüberzogene sowie überzogene Tabletten, z.B. magensaftresistente Überzüge), Dragees, Granulaten, Pellets, Feststoffmischungen, Dispersionen in flüssigen Phasen, als Emulsionen, als Pulver, als Lösungen, als Pasten oder als andere schluck- oder kaubare Zubereitungen vorliegen, z. B. als Nahrungsergänzungsmittel.

Die erfindungsgemäßen Halbfertigwaren dienen in der Regel zur Herstellung von (erfindungsgemäßen) der Ernährung oder dem Genuss dienenden gebrauchs- oder verzehrfertigen Zubereitungen.

Insbesondere können erfindungsgemäße Halbfertigwaren zur additiven Verstärkung des Umami-Geschmacks von Natriumglutamat-reduzierten Nahrungs- und Genussmitteln und auch direkt als Würzmittel für die industrielle oder nicht-industrielle Zubereitung von Nahrungs- und/oder Genussmitteln dienen.

Erfindungsgemäß besonders bevorzugt ist eine Halbfertigware (wie oben beschrieben), die bezogen auf das Gesamtgewicht der Halbfertigware eine Gesamtmenge an Natriumglutamat im Bereich von 0,00001 bis 10 Gew.-%, bevorzugt 0,0001 bis 5 Gew.-%, besonders bevorzugt von 0,001 bis 2 Gew.-%, oder kein Natriumglutamat umfasst.

Grundsätzlich enthalten erfindungsgemäß bevorzugte Zubereitungen oder Halbfertigwaren vorzugsweise:
- eine Gesamtmenge von 5 ppm bis 500.000 ppm, bevorzugt 25 ppm bis 250.000 ppm, insbesondere 10 ppm bis 10.000 ppm an erfindungsgemäßen aromatischen Alkensäurederivaten der Formel (I), insbesondere Verbindungen 1 bis 6,
- oder alternativ einen Anteil von 0,0001 Gew.-% bis 90 Gew.-%, bevorzugt 0,001 Gew.-% bis 30 Gew.-% einer erfindungsgemäßen Aromazubereitung
- kein Natriumglutamat oder einen Anteil von 0,00001 bis 10 Gew.-%, bevorzugt 0,0001 bis 5 Gew.-%, insbesondere 0,001 Gew.-% bis 2 Gew.-% an Natriumglutamat,
jeweils bezogen auf das Gesamtgewicht der Zubereitungen oder der Halbfertigware.

Die erfindungsgemäßen Zubereitungen oder Halbfertigwaren werden vorzugsweise hergestellt, indem die aromatischen Alkensäurederivate der Formel (I) in Ethanol und gegebenenfalls demineralisiertem und/oder gereinigtem Wasser gelöst bzw. gemischt werden. Anschließend werden die Lösungen durch einen Trocknungsprozess, vorzugsweise einen Sprühtrocknungs-, Vakuumgefriertrockungs-, Umkehrosmose-, Eindampf- oder anderen Konzentrationsprozess oder eine Kombination der genannten Prozesse, in eine (zumindest nahezu) feste Form überführt. Dabei kann die Trocknung unter Zuhilfenahme von Trägerstoffen (z.B. Stärke, Stärkederivate, Maltodextrin, Kieselgel, siehe oben) oder Hilfsstoffen (z.B. Pflanzengummen, Stabilisierungsmittel) erfolgen. Vorzugsweise erfolgt die Trocknung mittels Sprühtrocknung oder Vakuumgefriertrocknung.

Bevorzugte erfindungsgemäße Zubereitungen oder Halbfertigwaren sind Würze, Würzmischungen, Würzmittel, Brühwürfel, Instant-Suppen, Instant-Soßen, vegetarische Fertiggerichte, fleischhaltige Fertiggerichte, Fischsoßen wie z.B. Anchovis-Soßen, Austernsoßen und Sojasoßen.

Gemäß einer weiteren bevorzugten Ausführungsform werden zur Herstellung erfindungsgemäßer Zubereitungen oder Halbfertigwaren aromatische Alkensäurederivate der Formel (I) sowie gegebenenfalls andere Bestandteile zunächst in Emulsionen, in Liposomen (z.B. ausgehend von Phosphatidylcholin) in Microsphären, in Nanosphären oder auch in Kapseln, Granulate oder Extrudate aus einer für Lebens- und Genussmittel geeigneten Matrix (z.B. aus Stärke, Stärkederivaten, Cellulose oder Cellulosederivaten wie Hydroxypropylcellulose, anderen Polysacchariden wie Alginat, natürlichen Fetten, natürlichen Wachsen wie Bienenwachs oder Carnaubawachs oder aus Proteinen wie Gelatine) eingearbeitet.

In einem weiteren bevorzugten Herstellungsverfahren werden aromatische Alkensäurederivate der Formel (I) mit einem oder mehreren geeigneten Komplexbildnern, beispielsweise mit Cyclodextrinen oder Cyclodextrinderivaten, bevorzugt alpha- oder beta-Cyclodextrin, komplexiert und in dieser komplexierten Form eingesetzt.

Besonders bevorzugt sind erfindungsgemäße Zubereitungen oder Halbfertigwaren, bei denen die Matrix so gewählt ist, dass die aromatischen Alkensäurederivate der Formel (I) verzögert von der Matrix freigegeben werden, so dass man eine langanhaltende Wirkung erhält. Als Matrix können hier z.B. natürliche Fette, natürliche Wachse (z.B. Bienenwachs, Carnaubawachs) oder auch natürliche Ballaststoffe (Weizenfasern, Apfelfasern, Haferfasern, Orangenfasern) verwendet werden.

Im Zusammenhang mit der vorliegenden Erfindung wird auch ein Verfahren zum Vermitteln, Modifizieren und/oder Verstärken eines Umami-, Kokumi- und/oder salzigen Geschmacks einer der Ernährung oder dem Genuss dienenden gebrauchs- oder verzehrfertigen Zubereitung oder Halbfertigware beschrieben. Ein solches Verfahren umfasst den Schritt
(a) Vermischen einer Umami-geschmacklich wirksamen Menge (für bevorzugte Mengen s. oben), eines oder mehrerer erfindungsgemäß zu verwendender aromatischen Alken-säurederivate der Formel (I) oder einer erfindungsgemäßen Geschmacksstoffmischung (wie oben beschrieben) oder eines erfindungsgemäßen pflanzlichen Extrakts (wie oben beschrieben) mit einem oder mehreren weiteren Bestandteilen der Zubereitung bzw. Halbfertigware, oder
(b) Applizieren einer Umami-geschmacklich wirksamen Menge eines oder mehrerer aromatischen Alkensäurederivate der Formel (I) oder einer erfindungsgemäßen Aromamischung auf einen oder mehrere weitere Bestandteile der Zubereitung bzw. Halbfertigware, oder
(c) Einbetten einer geschmacklich wirksamen Menge eines oder mehrerer aromatischen Alkensäurederivate der Formel (I) oder einer erfindungsgemäßen Aromamischung in ein Hüll- oder Matrixmaterial.

### GEWERBLICHE ANWENDBARKEIT

Die in der vorliegenden Erfindung gefundenen aromatischen Alkensäurederivate der Formel (I) sind insbesondere geeignet für den Einsatz im Bereich Lebensmittel und Nahrungsmittelsektor. Hierbei werden die erfindungsgemäßen aromatischen Alkensäurederivate der Formel (I), insbesondere Verbindungen 1 bis 6 in Lebens-/Nahrungs- und Genussmitteln und / oder Halbfertigwaren, bevorzugt als Geschmackstoffe, zur Vermittlung oder Erzielung eines selbstständigen oder Verstärkung eines vorhandenen Umami- oder Kokumi-artigen oder salzigen Geschmackeffekts, eingesetzt.

Eine bevorzugte Ausführungsform der vorliegenden Erfindung sind daher orale Zubereitungen, umfassend
(a) mindestens eine erfindungsgemäße Aromamischung,
   oder
(b) mindestens ein Alkensäurederivate der allgemeinen Formel (I), vorzugsweise mindestens eines der Verbindungen 1 bis 6, bevorzugt Mischungen aus mindestens zwei, drei oder mehreren der Verbindungen 1 bis 6,
   und
(c) mindestens einen weiteren zum Verzehr geeigneten Bestandteil ausgewählt aus der Gruppe die gebildet wird von Trägerstoffen, Aromastoffen, Fetten und Ölen, Emulgatoren und Antioxidantien, weitere Hilfs- und Zusatzstoffe, Natriumglutamat, Zucker und Salz,
wobei es sich bei den Zubereitungen um eine der Ernährung oder dem Genuss dienende gebrauchs- oder verzehrfertige Zubereitungen oder entsprechende Halbfertigprodukte handelt.

In eine besonders bevorzugte Ausführungsform ist die orale Zubereitungen ausgewählt aus der Gruppe, die gebildet wird von Backwaren, Getränken, Instantgetränken, Fleischprodukten, gewürzten oder marinierten Fischprodukten, Eiern oder Eiprodukten Getreideprodukten, Milchprodukten Produkten aus Sojaprotein oder anderen Sojabohnen-Fraktionen, Gemüsezubereitungen, Knabberartikeln, Produkten auf Fett- und Ölbasis oder Emulsionen derselben, sonstigen Fertiggerichten, Suppen, Soßen, Gewürzen oder Gewürzzubereitungen sowie Aufstreuwürzungen.

In einer weiteren bevorzugten Ausführung liegt die Menge der Alkensäurederivate der allgemeinen Formel (I) in den oralen Zubereitungen im Bereich von 5 ppm bis 500 ppm, bevorzugt im Bereich von 10 bis 250 ppm, besonders bevorzugt von 10 ppm bis 100 ppm, bezogen auf das Gesamtgewicht der oralen Zubereitung.

### BEISPIELE

Die folgenden Anwendungsbeispiele dienen zur Verdeutlichung der Erfindung, ohne diese damit einzuschränken.

### Herstellungsbeispiel 1 bis 6

Die in dieser Anmeldung beschriebenen Stoffe können durch dem Fachmann vertraute chemische Verfahren ausgehend von den kommerziell erhältlichen Ausgangsrohstoffen Zimtsäure [140-10-3] beziehungsweise 3,4-(Methylenedioxy)zimtsäure [2373-80-0] dargestellt werden. In Schema 1 wird der gewählte Syntheseweg skizziert, allerdings sind weitere Herstellungswege möglich.

### Schema 1: Syntheseweg der Verbindungen 1 - 6

### Herstellbeispiel 1: (2E)-N-(Cinnamoyl)piperidine (Verbindung 1)

### Spektroskopische Daten und sensorische Beschreibung

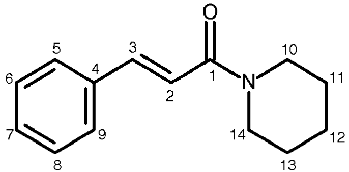

| **Position** | **¹H NMR 400 MHz, CD₃OD** | **¹³C NMR 100 MHz, CD₃OD** |
|---|---|---|
| | ***δ*_{H} (ppm), mult (*J* in Hz)** | ***δ*_{c} (ppm)** |
| 1 | | 165,4 C |
| 2 | 6,90 d (15,5) | 117,8 CH |
| 3 | 7,64 d (15,5) | 142,1 CH |
| 4 | | 135,5 C |
| 5/9 | 7,52 m | 127,7 CH |
| 6/8 | 7,35 m | 128,8 CH |
| 7 | 7,35 m | 129,4 CH |
| 10/14 | 3,63 d | 43,4; 47,0 CH2 |
| 11/13 | 1,62; 1,69 m | 25,6; 26,8 CH2 |
| 12 | 1,62; 1,69 m | 24,7 CH2 |

| **HR-MS (calc)** | **HR-MS** | **[*M*+H]⁺** | ***m*/*z*** | **UV λₘₐₓ (nm)** |
|---|---|---|---|---|
| 216,1383 | 216,1395 | C₁₄H₁₈NO | 103,0553; 131,0507 | 210, 220, 230, 280 |

Sensorische Daten: 50 ppm in Wasser: umami, scharf (verzögert)

### Herstellbeispiel 2: trans-Fagaramid (Verbindung 2)

### Spektroskopische Daten und sensorische Beschreibung

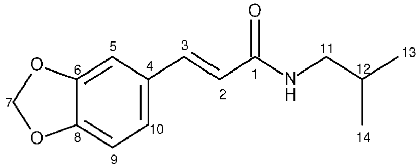

| **Position** | **¹H NMR 400 MHz, CDCl₃** | **¹³C NMR 100 MHz, CDCl₃** |
|---|---|---|
| | ***δ*_{H} (ppm), mult (*J* in Hz)** | ***δ*_{c} (ppm)** |
| 1 | | 166,1 C |
| 2 | 6,23 d (15,4) | 118,8 CH |
| 3 | 7,54 d (0,5; 15,4) | 140,7 CH |
| 4 | | 129,3 C |
| 5 | 7,00 m (0,5; 1,7) | 106,3 CH |
| 6 | | 149,0 C |
| 7 | 5,99 s | 101,4 CH2 |
| 8 | | 148,2 C |
| 9 | 6,79 d (0,4; 7,9) | 108,5 CH |
| 10 | 6,98 m (0,5; 1,7; 7,9) | 123,8 CH |
| 11 | 3,21 m (6,1; 6,8) | 47,1 CH2 |
| 12 | 1,84 m (6,7) | 28,7 CH |
| 13/14 | 0,95 d (6,7) | 20,2 CH3 |

| **HR-MS (calc)** | **HR-MS** | **[*M*+H]⁻** | ***m*/*z*** | **UV λₘₐₓ (nm)** |
|---|---|---|---|---|
| 248,1281 | 248,1291 | C₁₄H₁₇NO₃ | 175,0412; 89,0388 | 220, 230, 290, 320 |

Sensorische Daten: 50 ppm in Wasser: schwach Cumarin-artig, schwach Bittermandel, schwach brennend, umami, mundwässernd

### Herstellbeispiel 3: Ilepcimide (Verbindung 3)

### Spektroskopische Daten und sensorische Beschreibung

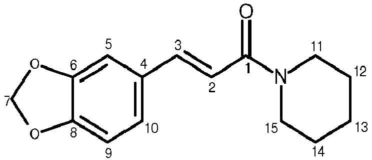

| **Position** | **¹H NMR 400 MHz, CDCl₃** | **¹³C NMR 100 MHz, CDCl₃** |
|---|---|---|
| | ***δ*_{H} (ppm), mult (*J* in Hz)** | ***δ*_{c} (ppm)** |
| 1 | | 165,4 C |
| 2 | 6,74 d (15,3) | 115,6 CH |
| 3 | 7,56 d (15,4) | 141,9 CH |
| 4 | | 129,9 C |
| 5 | 7,03 dt (0,5; 1,8) | 106,3 CH |
| 6 | | 148,2 C |
| 7 | 5,95 s | 101,4 CH2 |
| 8 | | 148,8 C |
| 9 | 6,79 d (8,1) | 108,5 CH |
| 10 | 6,99 ddd (0,6; 1,7; 8,0) | 123,6 CH |
| 11 | 3,62 m | 43,3 oder 47,0 CH2 |
| 12 | 1,64 m | 24,7 oder 25,6 oder 26,8 CH2 |
| 13 | 1,64 m | 24,7 oder 25,6 oder 26,8 CH2 |
| 14 | 1,64 m | 24,7 oder 25,6 oder 26,8 CH2 |
| 15 | 3,62 m | 43,3 oder 47,0 CH2 |

| **HR-MS (calc)** | **HR-MS** | **[*M*+H]⁺** | ***m*/*z*** | **UV λₘₐₓ (nm)** |
|---|---|---|---|---|
| 260,1281 | 260,1282 | C₁₅H₁₈NO₃ | 89,0382; 125,0299; 175,0408 | 220,230,290,320 |

Sensorische Daten: 50 ppm in Wasser: scharf (verzögert), irritierend, umami

### Herstellbeispiel 4: Dihydropiperlonguminin (Verbindung 4)

### Spektroskopische Daten und sensorische Beschreibung

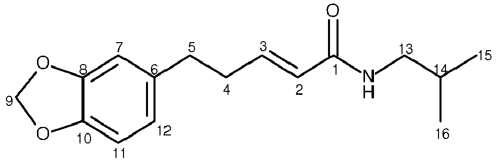

| **Position** | **¹H NMR 400 MHz, CDCl₃** | **¹³C NMR 100 MHz, CDCl₃** |
|---|---|---|
| | ***δ*_{H} (ppm), mult (*J* in Hz)** | ***δ*_{c} (ppm)** |
| 1 | | 165,8 C |
| 2 | 5,77 dt (1,5; 15,2) | 124,3 CH |
| 3 | 6,84 dt (6,9; 15,3) | 143,3 CH |
| 4 | 2,45 dtd (1,5; 7,0; 8,8) | 34,1 CH2 |
| 5 | 2,68 dd (6,8; 8,7) | 34,5 CH2 |
| 6 | | 134,9 C |
| 7 | 6,67 dd (0,5; 1,7) | 108,7 CH |
| 8 | | 147,6 C |
| 9 | 5,92 s | 100,8 CH2 |
| 10 | | 145,7 C |
| 11 | 6,72 d (7,9) | 108,3 CH |
| 12 | 6,62 ddt (0,6; 1,7; 7,8) | 121,2 CH |
| 13 | 3,14 dd (6,1; 6,9) | 46,9 CH2 |
| 14 | 1,79 dp (6,7; 13,4) | 28,6 CH |
| 15/16 | 0,92 d (6,7) | 20,1 CH3 |

| **HR-MS (calc)** | **HR-MS** | **[*M*+H]⁺** | ***m*/*z*** | **UV λₘₐₓ (nm)** |
|---|---|---|---|---|
| 276,1594 | 276,1588 | C₁₆H₂₂NO₃ | 135,0466 | 200, 215, 285 |

Sensorische Daten: 50 ppm in Wasser: bitter (langanhaltend), umami

### Herstelleispiel 5: Chingchengenamide A (Verbindung 5)

### Spektroskopische Daten und sensorische Beschreibung

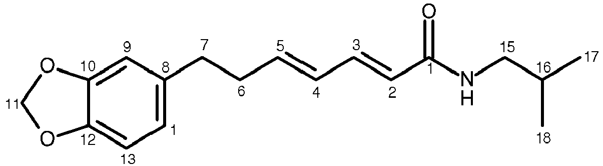

| **Position** | **¹H NMR 400 MHz, CD₃OD** | **¹³C NMR 100 MHz, CD₃OD** |
|---|---|---|
| | ***δ*_{H} (ppm), mult (*J* in Hz)** | ***δ*_{c} (ppm)** |
| 1 | | 169,2 C |
| 2 | 5,91 dt (0,6; 15,2) | 123,4 CH |
| 3 | 7,08 dd (10,4; 15,2) | 142,0 CH |
| 4 | 6,18 ddq (0,7; 1,2; 10,4; 15,2) | 130,4 CH |
| 5 | 6,09 dd (6,7; 15,2) | 142,8 CH |
| 6 | 2,43 q | 36,2 CH2 |
| 7 | 2,66 t | 36,0 CH2 |
| 8 | | 136,6 C |
| 9 | 6,69 dd (0,4; 1,7) | 109,8 CH |
| 10 | | 149,1 C |
| 11 | 5,88 s | 102,1 CH2 |
| 12 | | 147,2 C |
| 13 | 6,70 dd (0,5; 7,7) | 109,0 CH |
| 14 | 6,63 ddt (0,6; 1,8; 7,9) | 122,4 CH |
| 15 | 3,06 d (6,9) | 48,1 CH2 |
| 16 | 1,79 m (6,9) | 29,8 CH |
| 17/18 | 0,91 d (6,7) | 20,5 CH3 |

| **HR**-**MS (calc)** | **HR-MS** | **[*M*+H]⁺** | ***m*/*z*** | **UV λₘₐₓ (nm)** |
|---|---|---|---|---|
| 302,1751 | 302,1737 | C₁₈H₂₄NO₃ | 135,0454 | 200, 220, 260 |

Sensorische Daten: 50 ppm in Wasser: schwach süßlich, schwach umami

### Herestellbeispiel 6: (2E,4E)-Methyl-7-(1,3-benzodioxol-5-yl)hepta-2,4-dienoat (Verbindung 6)

### Spektroskopische Daten und sensorische Beschreibung

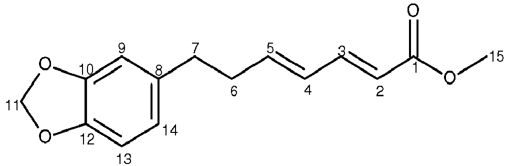

| **Position** | **¹H NMR 400 MHz, CD₃OD** | **¹³C NMR 100 MHz, CD₃OD** |
|---|---|---|
| | ***δ*_{H} (ppm), mult (*J* in Hz)** | ***δ*_{c} (ppm)** |
| 1 | | 169,3 C |
| 2 | 5,80 d (15,4) | 119,9 CH |
| 3 | 7,23 dd (9,8; 15,4) | 146,7 CH |
| 4 | 6,22 dd (9,8; 15,2) | 130,1 CH |
| 5 | 6,16 dt (6,4; 15,1) | 145,1 CH |
| 6 | 2,43 m | 35,8 CH2 |
| 7 | 2,65 m | 36,2 CH2 |
| 8 | | 136,4 C |
| 9 | 6,68 dd (0,5; 1,8) | 109,8 CH |
| 10 | | 149,1 C |
| 11 | 5,87 s | 102,1 CH2 |
| 12 | | 147,2 C |
| 13 | 6,70 dd (0,4; 8,0) | 109,0 CH |
| 14 | 6,62 ddt (0,6; 1,8; 7,9) | 122,3 CH |
| 15 | 3,70 s | 52,0 CH3 |

| **HR**-**MS (calc)** | **HR-MS** | **[*M*+H]⁺** | ***m*/*z*** | **UV λₘₐₓ (nm)** |
|---|---|---|---|---|
| 261,1121 | 261,1112 | C₁₅H₁₇O₄ | 128,0628; 135,0451 | 200, 260 |

Sensorische Daten: 50 ppm in Wasser: umami, schwach bitter, schwach salzig, schwach brennend

### Beispiel 7: Sprühtrocknung der erhaltenen Verbindungen aus Herstellbeispiel 1-6

Die Verbindungen aus Teil A werden gemischt, dann mit den Verbindungen des Teils B mit Hilfe eines Ultraturrax (IKA, Staufen) vermischt, in 250 ml wässrigem Ethanol gelöst und dann sprühgetrocknet (Inlet-Temperatur: 185-195°C, Outlet-Temperatur: 70-75°C, Büchi-Sprühtrockner B-290, Büchi, Flawil, Schweiz). Die resultierenden Pulver wurden in den nachfolgend beschriebenen Anwendungsbeispielen eingesetzt. Dazu wurde des Weiteren eine (nicht erfindungsgemäße) Aromakomposition mitverwendet, die 0,025 g *trans*-Pellitorin, 2.5 g Hesperitin, 1,5 g Phloretin und ad 100 g 1,2-Propylenglykol enthielt.

**Tabelle 1: Zusammensetzung der sprühgetrockneten Aromamischung I-X**

| **Tel** | **Bestandteil** | **Einsatz [Gew.%]** | | | | | | | | | |
|---|---|---|---|---|---|---|---|---|---|---|---|
| | | **I** | **II** | **III** | **IV** | **V** | **VI** | **VII** | **VIII** | **IX** | **X** |
| A | Verbindung 1 | 5 | 5 | | | | | | | | 1 |
| | Verbindung 2 | | 5 | 5 | | | | | | | 1 |
| | Verbindung 3 | 5 | | 5 | | | | | | 2,5 | 2 |
| | Verbindung 4 | | | | 10 | | | | | 2,5 | 2 |
| | Verbindung 5 | | | | | 10 | 5 | | 5 | | 2 |
| | Rubemamin | | | | | | 5 | 10 | | 2,5 | 1 |
| | Rubescenamin | | | | | | | | 5 | 2,5 | 1 |
| B | Maltodextrin | 90 | 90 | 90 | 90 | 80 | 80 | 70 | 90 | 90 | 90 |
| | Gummi arabicum | | | | | 10 | 10 | 20 | | | |

### Beispiel 8: Würzmittel

Teil A wurde eingewogen. Es wurden 290 ml Wasser vorgelegt, unter Rühren Teil A zugegeben und gelöst. Die Lösung wurde mit Wasser auf 1,84 kg verdünnt (pH 6,0) und anschließend gefriergetrocknet (Eutektischer Punkt: -15°C; Arbeitsvakuum: 0,52 mbar; Stellflächentemperatur: -5°C bis +25°C). Das Produkt wurde dann mit Senfsamenmehl und dem Senfaroma aus Teil B gemischt und zu einem Würzmittel konfektioniert.

**Tabelle 2: Zusammensetzung Würzmittel XI bis XVIII**

| **Teil** | **Bestandteil** | **Einsat (g)** | | | | | | | |
|---|---|---|---|---|---|---|---|---|---|
| | | **XI** | **XII** | **XIII** | **XIV** | **XV** | **XVI** | **XVII** | **XVIII** |
| A | Verbindung 1 | 0,2 | | | | | 0,1 | 0,1 | |
| | Verbindung 2 | | 0,2 | | | | | | 0,1 |
| | Verbindung 3 | | | 0,1 | | | | | |
| | Verbindung 4 | | | | 0,2 | | | 0,1 | |
| | Verbindung 5 | | | | | 0,2 | 0,1 | | |
| | Rubemamin | | | | | | 0,1 | 0,1 | |
| | Rubescenamin | | | 0,1 | 0,1 | | 0,1 | | 0,1 |
| | Natriumchlorid | 15 | 15 | 15 | 15 | 15 | 15 | 15 | 15 |
| B | Senfsamenmehl | 5 | 5 | 5 | 5 | 5 | 5 | 5 | 5 |
| | Senfaroma | 0,1 | 0,1 | 0,1 | 0,1 | 0,1 | 0,1 | 0,1 | 0,1 |

### Beispiel 9: Aromamischungen

Alle Komponenten werden bei 40°C gemischt und anschließend bei 85°C für 10 Minuten erhitzt (Rückflussreaktion). Nach dem Abkühlen auf 40°C wird mit Kalilauge auf pH 5 eingestellt.

**Tabelle 3: Zusammensetzung der Aromamischungen XIX bis XXIII**

| **Inhaltsstoff** | **Einsatz [g]** | | | | |
|---|---|---|---|---|---|
| | **XIX** | **XX** | **XX** | **XXII** | **XXII** |
| L-Alanin | 41,0 | 41,0 | 41,0 | 41,0 | 41,0 |
| L-Asparaginsäure | 123,0 | 123,0 | 123,0 | 123,0 | 123,0 |
| Bernsteinsäure | 4,7 | 4,7 | 4,7 | 4,7 | 4,7 |
| Calciumchlorid Dihydrat | 7,0 | 7,0 | 7,0 | 7,0 | 7,0 |
| L-Cystein•HCl Monohydrat | 11,0 | 11,0 | 11,0 | 11,0 | 11,0 |
| Dikaliumphosphat | 6,0 | 6,0 | 6,0 | 6,0 | 6,0 |
| Fructose gemahlen | 1,0 | 1,0 | 1,0 | 1,0 | 1,0 |
| L-Isoleucin | 1,6 | 1,6 | 1,6 | 1,6 | 1,6 |
| Kaliumchlorid | 228,0 | 228,0 | 228,0 | 228,0 | 228,0 |
| L-Leucin | 1,6 | 1,6 | 1,6 | 1,6 | 1,6 |
| L-Lysin•HCl | 3,6 | 3,6 | 3,6 | 3,6 | 3,6 |
| Magnesiumchlorid Hexahydrat | 19,0 | 19,0 | 19,0 | 19,0 | 19,0 |
| Maltodextrin | 49,0 | 49,0 | 49,0 | 49,0 | 49,0 |
| L-Phenylalanin | 2,0 | 2,0 | 2,0 | 2,0 | 2,0 |
| L-Prolin | 74,0 | 74,0 | 74,0 | 74,0 | 74,0 |
| L-Serin | 6,5 | 6,5 | 6,5 | 6,5 | 6,5 |
| L-Threonin | 3,0 | 3,0 | 3,0 | 3,0 | 3,0 |
| L-Valin | 9,0 | 9,0 | 9,0 | 9,0 | 9,0 |
| Verbindung 1 | 25,0 | | 5,0 | | 5,0 |
| Verbindung 2 | | 20,0 | | | |
| Verbindung 3 | | | 10,0 | | |
| Verbindung 4 | | | | 10,0 | |
| Verbindung 5 | | | | | 5,0 |
| Wasser | Ad 1000 | | | | |

### Anwendungsbeispiel 10: Instantsuppe, Typ Lauch-Creme

5 g der jeweiligen Pulvermischungen A, A1- A5 werden mit je 100 ml heißem Wasser aufgegossen, um eine verzehrfertige Suppe zu erhalten.

Formulierung A dient zum Vergleich, die Formulierungen A1, A2 und A3 sind erfindungsgemäß (Natriumglutamat-frei), die Formulierungen A4 und A5 sind Natriumglutamat-frei und salzreduziert.

**Tabelle 4: Zusammensetzung von Instantsuppen A, A1-A5**

| **Bestandteil** | **Einsatz [g]** | | | | | |
|---|---|---|---|---|---|---|
| | **A** | **A1** | **A2** | **A3** | **A4** | **A5** |
| Kartoffelstärke | 20,0 | 20,0 | 20,0 | 20,0 | 20,0 | 20,0 |
| Fettpulver | 25,0 | 25,0 | 25,0 | 25,0 | 25,0 | 25,0 |
| Lactose | 20,0 | 20,0 | 20,0 | 20,0 | 20,0 | 20,0 |
| Maltodextrin | 11,730 | 14,728 | 14,709 | 14,680 | 15,715 | 15,715 |
| Kochsalz | 8,0 | 8,0 | 8,0 | 8,0 | 7,0 | 7,0 |
| Natriumglutamat | 3,0 | - | - | - | - | - |
| Spinatpulver | 2,0 | 2,0 | 2,0 | 2,0 | 2,0 | 2,0 |
| Grünes Lauchpulver | 2,0 | 2,0 | 2,0 | 2,0 | 2,0 | 2,0 |
| Zitronensäure, als Pulver | 0,3 | 0,3 | 0,3 | 0,3 | 0,3 | 0,3 |
| Gehärtetes Pflanzenfett | 3,0 | 3,0 | 3,0 | 3,0 | 3,0 | 3,0 |
| Gefriergetrockneter Lauch | 1,0 | 1,0 | 1,0 | 1,0 | 1,0 | 1,0 |
| Huhn-Aroma | 1,0 | 1,0 | 1,0 | 1,0 | 1,0 | 1,0 |
| Würze Typ "grüner Lauch", Pulver | 2,0 | 2,0 | 2,0 | 2,0 | 2,0 | 2,0 |
| Würze, Typ "gekochte Zwiebel" | 0,6 | 0,6 | 0,6 | 0,6 | 0,6 | 0,6 |
| Hefewürze, Typ "Gemüsebrühe" | 0,3 | 0,3 | 0,3 | 0,3 | 0,3 | 0,3 |
| Curcuma-Extrakt | 0,07 | 0,07 | 0,09 | 0,07 | 0,07 | 0,07 |
| Verbindung 1 | - | 0,025 | | | | |
| Verbindung 2 | | | 0,015 | | | |
| Verbindung 3 | | | | 0,050 | | |
| Verbindung 4 | | | | | 0,055 | |
| Verbindung 5 | | | | | | 0,075 |

### Anwendungsbeispiel 11: Instantsuppe, Typ Hühnersuppe mit Nudeln

4,6 g der jeweiligen Pulvermischungen B und B1 bis B4 werden 10 Minuten in je 100 ml Wasser gekocht, um eine verzehrfertige Suppe zu erhalten.

Formulierung B dient zum Vergleich, die Formulierungen B1, B2 und B3 sind erfindungsgemäß (Natriumglutamat-frei), die Formulierung B4 ist Natriumglutamat-frei und salzreduziert.

**Tabelle 5: Zusammensetzung von Instantsuppen B, B1-B4**

| **Bestandteil** | **Einsatz [g]** | | | | |
|---|---|---|---|---|---|
| | **B** | **B1** | **B2** | **B3** | **B4** |
| Stärke | 16,0 | 16,0 | 16,0 | 16,0 | 16,0 |
| Kochsalz | 7 | 7 | 7 | 7 | 5 |
| Saccharose, raffiniert | 3,2 | 3,2 | 3,2 | 3,2 | 3,2 |
| Natriumglutamat | 3,2 | - | - | - | - |
| Natriuminosinat / Natriumguanylat im Verhältnis 1:1 | 0,8 | 0,8 | 0,8 | 0,8 | 0,8 |
| Säurehydrolysiertes Pflanzenprotein | 8,0 | 8,0 | 8,0 | 8,0 | 8,0 |
| Fettpulver | 2,0 | 2,0 | 2,0 | 2,0 | 2,0 |
| Gemüsefett, sprühgetrocknet | 1,0 | 1,0 | 1,0 | 1,0 | 1,0 |
| Gefriergetrocknetes Hühnerfleisch, in Stückchen | 2,0 | 2,0 | 2,0 | 2,0 | 2,0 |
| Suppen-Nudeln | 32,0 | 32,0 | 32,0 | 32,0 | 32,0 |
| Chinesisches Gemüse, gefriergetrocknet | 4,6 | 4,6 | 4,6 | 4,6 | 4,6 |
| Huhn-Aroma | 8,0 | 8,0 | 8,0 | 8,0 | 8,0 |
| Lebensmittelfarbstoff Riboflavin | 0.04 | 0.04 | 0.04 | 0.04 | 0.04 |
| Würzmittel I (Beispiel 7) | - | 1,2 | - | - | - |
| Würzmittel VI (Beispiel 7) | - | - | 1,2 | - | - |
| Würzmittel IX (Beispiel 7) | - | - | - | 1,2 | - |
| Würzmittel X (Beispiel 7) | - | - | - | - | 1,2 |
| Maltodextrin | Ad 100 | Ad 100 | Ad 100 | Ad 100 | Ad 100 |

### Anwendungsbeispiel 12: Würzmischung Typ Pfeffer

Jeweils 100 g Nackensteak vom Schwein werden mit jeweils 1,7 g der Zubereitungen C, C1-C4 gleichmäßig bestreut und gebraten.

Formulierung C dient zum Vergleich, die Formulierungen C1, C2 und C3 sind erfindungsgemäß (Natriumglutamat-frei), die Formulierung C4 ist Natriumglutamat-frei und salzreduziert.

**Tabelle 6: Zusammensetzung von Würzmischungen C, C1-C4**

| **Bestandteil** | **Einsatz [g]** | | | | |
|---|---|---|---|---|---|
| | **C** | **C1** | **C2** | **C3** | **C4** |
| Milchprotein | 0,8 | 0,8 | 0,8 | 0,8 | 0,8 |
| Johannisbrotkernmehl | 2,0 | 2,0 | 2,0 | 2,0 | 2,0 |
| Kochsalz | 14,0 | 14,0 | 14,0 | 14,0 | 12,0 |
| Paprikapulver | 13,0 | 13,0 | 13,0 | 13,0 | 13,0 |
| Tomatenpulver | 13,0 | 13,0 | 13,0 | 13,0 | 13,0 |
| Saccharose | 4,0 | 4,0 | 4,0 | 4,0 | 4,0 |
| Knoblauchpulver | 0,5 | 0,5 | 0,5 | 0,5 | 0,5 |
| Gehärtetes Pflanzenfett | 8,0 | 8,0 | 8,0 | 8,0 | 8,0 |
| Fettpulver | 11,0 | 11,0 | 11,0 | 11,0 | 11,0 |
| Natriumglutamat | 6,0 | - | - | - | - |
| Lebensmittelfarbstoff Rote Bete und Paprika | 2,0 | 2,0 | 2,0 | 2,0 | 2,0 |
| Aroma Typ "Pfeffer" | 2,0 | 2,0 | 2,0 | 2,0 | 2,0 |
| Aroma Typ "Pizza" | 1,2 | 1,2 | 1,2 | 1,2 | 1,2 |
| Aroma Typ "Tomate" | 0,4 | 0,4 | 0,4 | 0,4 | 0,4 |
| Extrakt aus schwarzem Pfeffer | 0,1 | 0,1 | 0,1 | 0,1 | 0,1 |
| Rubemamin | - | 0,005 | - | - | 0,05 |
| Verbindung 1 | 0,03 | - | 0,1 | 0,01 | 0,1 |
| Verbindung 2 | 0,05 | - | - | 0,04 | - |
| Verbindung 3 | - | 0,05 | - | 0,01 | - |
| Verbindung 4 | - | 0,05 | - | 0,03 | 0,1 |
| Verbindung 5 | 0,2 | 0,05 | - | 0,06 | 0,1 |
| Maisstärke | Ad 100 | Ad 100 | Ad 100 | Ad 100 | Ad 100 |

### Anwendungsbeispiel 13: Tomatenketchup

Die Inhaltsstoffe werden in der angegebenen Reihenfolge gemischt und der fertige Ketchup mit Hilfe eines Rührwerkes homogenisiert, in Flaschen abgefüllt und sterilisiert.

Die Formulierungen D und D1 dienen zum Vergleich, die Formulierungen D2 (Natriumglutamat-frei, Zucker-reduziert) und D3 (Salz- und Zucker-reduziert; Natriumglutamat-frei) sind erfindungsgemäß.

**Tabelle 7: Zusammensetzung von Tomatenketchup D, D1-D3**

| **Bestandteil** | **Einsatz [g]** | | | |
|---|---|---|---|---|
| | **D** | **D1** | **D2** | **D3** |
| Natriumglutamat | 0,40 | - | - | - |
| Kochsalz | 2 | 1 | 2 | 1 |
| Stärke, Farinex WM 55 | 1 | 1 | 1 | 1 |
| Sucrose | 12 | 9,2 | 9,2 | 9,2 |
| Tomaten-Konzentrat 2-fach | 40 | 40 | 40 | 40 |
| Glucosesirup 80 Brix | 18 | 18 | 18 | 18 |
| Branntweinessig 10% | 7 | 7 | 7 | 7 |
| Wasser | 19,60 | 23,80 | 22,30 | 23,25 |
| Würzmittel IX (Beispiel 7) | - | - | 0,08 | 0,02 |
| 5 %ige Lösung der Verbindung 1 in Propylenglycol | - | - | 0,10 | - |
| 5 %ige Lösung der Verbindung 5 in Propylenglycol | - | - | - | 0,15 |

### Anwendungsbeispiel 14: Bouillon

15 g der jeweiligen Pulvermischungen E, E1-E4 werden mit je 1000 ml heißem Wasser aufgegossen.

Die Formulierungen E und E1 dienen zum Vergleich, die Formulierungen E2, E3 (Natriumglutamat-frei) und E4 (Salz-reduziert; Natriumglutamat-frei) sind erfindungsgemäß.

**Tabelle 8: Zusammensetzung von Bouillon E, E1-E4**

| **Bestandteil** | **Einsatz [g]** | | | | |
|---|---|---|---|---|---|
| | **E** | **E1** | **E2** | **E3** | **E4** |
| Fettpulver | 8,77 | 8,77 | 8,77 | 8,77 | 8,77 |
| Natriumglutamat | 8,77 | 5 | 5 | - | - |
| Hefeextrakt Pulver | 12,28 | 12,28 | 12,28 | 12,28 | 12,28 |
| Kochsalz | 29,83 | 29,83 | 29,83 | 29,83 | 26,83 |
| Maltodextrin | 37,28 | 41,05 | 40,65 | 45,75 | 48,95 |
| Natürlicher Gemüseextrakt | 3,07 | 3,07 | 3,07 | 3,07 | 3,07 |
| Verbindung 1 | - | - | 0,08 | 0,30 | 0,05 |
| Verbindung 2 | - | - | 0,08 | - | - |
| Verbindung 3 | - | - | 0,08 | - | - |
| Verbindung 4 | - | - | 0,08 | - | - |
| Verbindung 5 | - | - | 0,08 | - | 0,05 |

### Anwendungsbeispiel 15: Würzmischung für Kartoffelchips

6 g der Würzmischungen F, F1-F4 werden auf 94 g Kartoffelchips aufgezogen.

Die Formulierungen F und F1 dienen zum Vergleich, die Formulierungen F2 (Natriumglutamat-reduziert), F3 (Natriumglutamat-frei) und F4 (Salz-reduziert; Natriumglutamat-frei) sind erfindungsgemäß.

**Tabelle 9: Zusammensetzung von Würzmischungen F, F1-F4 für Kartoffelchips**

| **Bestabdteil** | **Einsatz [g]** | | | | |
|---|---|---|---|---|---|
| | **F** | **F1** | **F2** | **F3** | **F4** |
| Natriumglutamat | 3,50 | 2,00 | 2,00 | - | 1,00 |
| Käsepulver | 10,00 | 10,00 | 10,00 | 10,00 | 10,00 |
| Knoblauchpulver | 2,00 | 2,00 | 2,00 | 2,00 | 2,00 |
| Molkenpulver | 38,86 | 40,36 | 40,06 | 41,91 | 44,76 |
| Würzextraktöl | 0,20 | 0,20 | 0,20 | 0,20 | 0,20 |
| Paprikapulver | 9,80 | 9,80 | 9,80 | 9,80 | 9,80 |
| Kochsalz | 21,00 | 21,00 | 21,00 | 21,00 | 17,00 |
| Tomatenpulver | 9,00 | 9,00 | 9,00 | 9,00 | 9,00 |
| Trockenaroma | 2,50 | 2,50 | 2,50 | 2,50 | 2,50 |
| Siliciumdioxid | 0,02 | 0,02 | 0,02 | 0,02 | 0,02 |
| Pflanzenöl | 0,02 | 0,02 | 0,02 | 0,02 | 0,02 |
| Zwiebelpulver | 3,00 | 3,00 | 3,00 | 3,00 | 3,00 |
| Sahne Aromakonzentrat | 0,03 | 0,03 | 0,03 | 0,03 | 0,03 |
| Käse Aroma | 0,03 | 0,03 | 0,03 | 0,03 | 0,03 |
| Tomaten Aromakonzentrat | 0,04 | 0,04 | 0,04 | 0,04 | 0,04 |
| Sprühgetrocknete Aromamischung I (Beispiel 7) | - | - | 1,30 | - | - |
| Sprühgetrocknete Aromamischung VI (Beispiel 7) | - | - | - | 2,45 | - |
| Sprühgetrocknete Aromamischung X (Beispiel 7) | - | - | - | - | 1,60 |

### Anwendungsbeispiel 16: Weiße Sauce

90 g der Soßenmischungen G, G1-G4 werden mit 1000 ml heißem Wasser aufgegossen und mit dem Schneebesen kräftig verrührt.

Die Formulierungen G und G1 dienen zum Vergleich, die Formulierungen G2 (Natriumglutamat-reduziert),G3 (Natriumglutamat-frei) und G4 (Salz-reduziert; Natriumglutamat-frei) sind erfindungsgemäß.

**Tabelle 10: Zusammensetzung von weißen Saucen G, G1-G4**

| **Bestandteil** | **G** | **G1** | **G2** | **G3** | **G4** |
|---|---|---|---|---|---|
| Maltodextrin | 25,98 | 27,18 | 27,08 | 27,58 | 28,43 |
| Kochsalz | 7,50 | 7,50 | 7,50 | 7,50 | 6,00 |
| Natriumglutamat | 2,00 | 0,80 | 0,80 | - | 0,80 |
| Pflanzenfett | 5,00 | 5,00 | 5,00 | 5,00 | 5,00 |
| Pfeffer, weiß | 0,02 | 0,02 | 0,02 | 0,02 | 0,02 |
| Zwiebelpulver | 1,50 | 1,50 | 1,50 | 1,50 | 1,50 |
| vorverkleisterte Maisstärke | 30,00 | 30,00 | 30,00 | 30,00 | 30,00 |
| Fettpulver | 28,00 | 28,00 | 28,00 | 28,00 | 28,00 |
| Sprühgetrocknete Aromamischung II (Beispiel 7) | - | - | 1,1 | - | - |
| Sprühgetrocknete Aromamischung V (Beispiel 7) | - | - | - | 1,1 | - |
| Sprühgetrocknete Aromamischung IX (Beispiel 7) | - | - | - | - | 2,3 |

### Anwendungsbeispiel 17: Braune Sauce

90 g der Soßenmischungen H, H1-H4 werden mit 1000 ml heißem Wasser aufgegossen und mit dem Schneebesen kräftig verrührt.

Die Formulierungen H und H1 dienen zum Vergleich, die Formulierungen H2 (Natriumglutamat-reduziert), H3 (Natriumglutamat-frei) und H4 (Salz-reduziert; Natriumglutamat-frei) sind erfindungsgemäß.

**Tabelle 11: Zusammensetzung von braunen Saucen H, H1-H4**

| **Bestandeil** | **Einsatz [g]** | | | | |
|---|---|---|---|---|---|
| | **H** | **H1** | **H2** | **H3** | **H4** |
| Stärke | 40,00 | 40,00 | 40,00 | 40,00 | 40,00 |
| Maltodextrin | 33,1 | 33,8 | 32,7 | 33,9 | 33,2 |
| Kochsalz | 6,00 | 6,00 | 6,00 | 6,00 | 4,50 |
| Zuckerkulör, sprühgetrocknet | 5,00 | 5,00 | 5,00 | 5,00 | 5,00 |
| Hefeextraktpulver | 3,00 | 3,00 | 3,00 | 3,00 | 3,00 |
| Natriumglutamat | 2,00 | 1,30 | 1,30 | - | 1,30 |
| Zucker | 0,50 | 0,50 | 0,50 | 0,50 | 0,50 |
| Fettpulver | 5,00 | 5,00 | 5,00 | 5,00 | 5,00 |
| Tomatenpulver | 3,00 | 3,00 | 3,00 | 3,00 | 3,00 |
| Natürlicher Gemüseextrakt | 1,00 | 1,00 | 1,00 | 1,00 | 1,00 |
| Zwiebelextrakt | 0,30 | 0,30 | 0,30 | 0,30 | 0,30 |
| Pfefferextrakt | 0,10 | 0,10 | 0,10 | 0,10 | 0,10 |
| Trockenaroma | 1,00 | 1,00 | 1,00 | 1,00 | 1,00 |
| Sprühgetrocknete Aromamischung III (Beispiel 7) | - | - | 1,1 | - | - |
| Sprühgetrocknete Aromamischung IV (Beispiel 7) | - | - | - | 1,2 | - |
| Sprühgetrocknete Aromamischung VII (Beispiel 7) | - | - | - | - | 2,1 |

### Anwendungsbeispiel 18: Tomatensuppe

Die festen Bestandteile werden eingewogen, gemischt und dem Wasser hinzugefügt. Das Pflanzenöl wird zudosiert und die Tomatenpaste hinzugegeben. Die Mischung wird unter Rühren aufgekocht.

Die Formulierungen I und I1 dienen zum Vergleich, die Formulierungen I2 (Natriumglutamat-reduziert), I3 (Natriumglutamat-frei) und I4 (Salz-reduziert; Natriumglutamat-frei) sind erfindungsgemäß.

**Tabelle 12: Zusammensetzung von Tomatensuppe I, 11-14**

| **Bestandteil** | **Einsatz [g]** | | | | |
|---|---|---|---|---|---|
| | **I** | **I1** | **I2** | **I3** | **I3** |
| Wasser | 50,65 | 50,80 | 50,79 | 50,90 | 51,20 |
| Pflanzenöl | 5,50 | 5,50 | 5,50 | 5,50 | 5,50 |
| Tomatenpaste | 24,00 | 24,00 | 24,00 | 24,00 | 24,00 |
| Sahne | 1,05 | 1,05 | 1,05 | 1,05 | 1,05 |
| Zucker | 2,00 | 2,00 | 2,00 | 2,00 | 2,00 |
| Kochsalz | 1,70 | 1,70 | 1,70 | 1,70 | 1,20 |
| Natriumglutamat | 0,40 | 0,25 | 0,25 | - | 0,25 |
| Weizenmehl | 5,50 | 5,50 | 5,50 | 5,50 | 5,50 |
| Stärke | 1,20 | 1,20 | 1,20 | 1,20 | 1,20 |
| gewürfelte Tomaten | 8,00 | 8,00 | 8,00 | 8,00 | 8,00 |
| Sprühgetrocknete Aromamischung V (Beispiel 7) | - | - | 0,01 | - | - |
| Sprühgetrocknete Aromamischung IX (Beispiel 7) | - | - | - | 0,15 | - |
| Sprühgetrocknete Aromamischung X (Beispiel 7) | - | - | - | - | 0,10 |

### Anwendungsbeispiel 19: Grünteegetränk

Das Grünteekonzentrat wird mit der jeweiligen 5 %igen Lösung des erfindungsgemäß zu verwendenden aromatischen Alkensäurederivats der Formel (I) in Propylenglycol vermischt. Anschließend wird mit entmineralisiertem Wasser aufgefüllt und erneut gründlich durchmischt. Dann wird das Produkt gefiltert, verbrauchsfertig verpackt und bei 118°C sterilisiert.

**Tabelle 13: Zusammensetzung von Grünteegetränken J, J1-J4**

| **Inhaltsstoff** | **Einsatz [Gew.%]** | | | | |
|---|---|---|---|---|---|
| | **J** | **J1** | **J2** | **J3** | **J4** |
| Grünteekonzentrat | 18.00 | 18.00 | 18.00 | 18.00 | 18.00 |
| 5% ige Lösung Rubemamin in Propylenglycol | | 0.002 | 0.001 | 0.001 | |
| 5% ige Lösung Verbindung 1 in Propylenglycol | 0,02 | | 0,01 | | |
| 5% ige Lösung Verbindung 2 in Propylenglycol | | 0,02 | 0,01 | | |
| 5% ige Lösung Verbindung 3 in Propylenglycol | | | 0,01 | 0,03 | |
| 5% ige Lösung Verbindung 4 in Propylenglycol | | | 0,01 | | |
| 5% ige Lösung Verbindung 5 in Propylenglycol | | | 0,01 | 0,02 | 0,04 |
| entmineralisiertes Wasser | Ad 100 | Ad 100 | Ad 100 | Ad 100 | Ad 100 |

### Anwendungsbeispiel 20: Rindfleischwürzmischung für (Fertig-Nudeln)

Alle Inhaltsstoffe in Tabelle 14 werden vermischt, bis sich eine homogene Mischung ergibt.

**Tabelle 14: Zusammensetzung von Rindfleischwürzmischungen**

| **Ihnatsstoff** | **Einsatz [Gew.%]** |
|---|---|
| Rindsfettaroma | 5,00 |
| Zuckercouleur | 3.00 |
| Zitronensäure (wasserfrei) | 0.40 |
| Schnittlauch (entwässert) | 2.00 |
| Maltodextrin (ex Tapioka) | 10.30 |
| Mononatriumglutamat | 15.00 |
| Zwiebelpulver | 5.00 |
| Ribotide | 0.80 |
| Natriumchlorid | 45.7 |
| Zucker | 2.80 |
| Süßmolkepulver | 6.50 |
| 10 %ige Lösung der Verbindung 2 in Propylenglykol | 3.5 |

### Anwendungsbeispiel 21: Nudeln

Eine Suspension der Zutaten B in Wasser wird zu einer Mischung der Zutaten A gegeben und zu einem Teig geknetet. Nachdem der Teig für ca. 5 Minuten geruht hat, wird dieser mit Hilfe einer Nudelmaschine zu Platten verarbeitet, die in einem letzten Arbeitsschritt in eine übliche Form zurechtgeschnitten werden. Die Nudeln sind nach einer Kochzeit von 3 Minuten verzehrfertig und können z. B. mit 8 g der Rindfleischwürzmischung (Anwendungsbeispiel 12) angerichtet werden.

**Tabelle 15: Zusammensetzung von Nudeln**

| **Teil** | **Inhaltsstoff** | **Einsatz [Gew.%]** |
|---|---|---|
| A | Weizenmehl | 62.00 |
| | Kartoffelstärke | 10.90 |
| B | Salz | 1.10 |
| | Guarkernmehl | 0.06 |
| | Natriumcarbonat | 0.07 |
| | Kaliumcarbonat | 0.25 |
| | Na₂H₂P₂O₇ | 0.07 |
| | 10 %ige Lösung einer 1:1-Mischung der Verbindungen 2 und 4 in Propylenglykol | 0.08 |
| C | Wasser | Ad 100 |

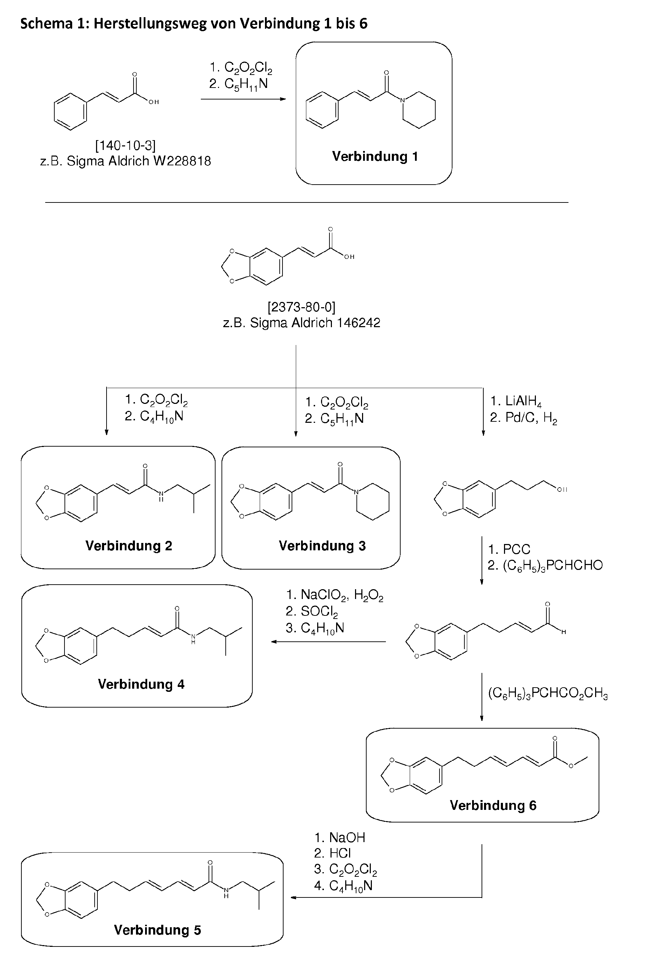

## Patentansprüche

1. Aromatische Alkensäurederivate der Formel (I) 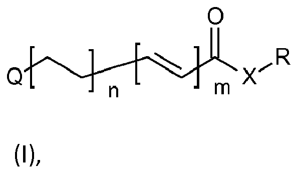 wobei Q einen Rest 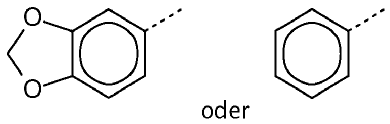 darstellt,
und entweder
i) n einen Wert 0 und gleichzeitig m einen Wert 1 oder
ii) n einen Wert 1 und m gleichzeitig einen Wert 1 oder 2 darstellt, und
wobei die resultierende(n) Doppelbindung(en) unabhängig voneinander als E-oder Z-Konfiguration vorliegen, und
X = - OR¹ oder - NR²R³ bedeutet, mit
R¹ = Wasserstoff, Methyl, Ethyl, Propyl, 2-Propyl, 1-Butyl, 2-Butyl, 2-Methyl-1-propyl, 1-Pentyl, 2-Pentyl, 3-Pentyl, 2-Methyl-1-butyl, 2-Methyl-2-butyl, 2-Methyl-3-butyl, 3-Methyl-1-butyl, 1-Hexyl, 2-Hexyl, 3-Hexyl, 2-Methyl-1-pentyl, 2-Methyl-2-pentyl, 2-Methyl-3-pentyl, 2-Methyl-4-pentyl, 3-Methyl-1-pentyl, 3-Methyl-2-pentyl oder 3-Methyl-3-pentyl, Allyl, Prenyl oder Isoprenyl,
und wobei
R², R³ unabhängig voneinander ausgewählt ist aus der Gruppe bestehend aus: Wasserstoff, Methyl, Ethyl, Propyl, 2-Propyl, 1-Butyl, 2-Butyl, 2-Methyl-1-propyl, 1-Pentyl, 2-Pentyl, 3-Pentyl, 2-Methyl-1-butyl, 2-Methyl-2-butyl, 2-Methyl-3-butyl, 3-Methyl-1-butyl, 1-Hexyl, 2-Hexyl, 3-Hexyl, 2-Methyl-1-pentyl, 2-Methyl-2-pentyl, 2-Methyl-3-pentyl, 2-Methyl-4-pentyl, 3-Methyl-1-pentyl, 3-Methyl-2-pentyl oder 3-Methyl-3-pentyl, Allyl, Prenyl, Isoprenyl,
oder
R² und R³ zusammen einen gesättigten oder ungesättigten carbocyclischen Ring ausbilden, ausgewählt aus der Gruppe bestehend aus: -CH2-CH2-, -CH2-CH2-CH2-,-CH2-CH2-CH2-CH2-, -CH=CH-CH=CH-, -CH2-CH2-CH2-CH2-CH2-, -CH=CH-CH2-CH2-CH2-, -CH2-CH=CH-CH2-CH2-, -CH=CH-CH=CH-CH2-,-CH=CH-CH=CH-CH=.

2. Aromatische Alkensäurederivate der Formel (I) nach Anspruch 1, wobei Q einen Rest 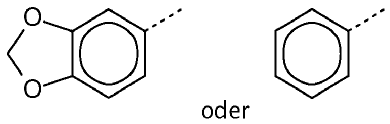 darstellt,
und entweder
i) n einen Wert 0 und gleichzeitig m einen Wert 1 oder
ii) n einen Wert 1 und m gleichzeitig einen Wert 1 oder 2 darstellt, und
wobei die resultierende(n) Doppelbindung(en) unabhängig voneinander als E-oder Z-Konfiguration vorliegen, und
X = - OR¹ mit
R¹= Wasserstoff, Methyl, Ethyl, Propyl, 2-Propyl, 1-Butyl, 2-Butyl, 2-Methyl-1-propyl oder - NR²R³bedeutet,
und wobei
R² Wasserstoff ist
und gleichzeitig
R³ Methyl, Ethyl, 2-Methyl-1-propyl, 2-Methyl-1-butyl, Allyl, Prenyl, Isoprenyl
oder
R² und R³ zusammen einen gesättigten oder ungesättigten carbocyclischen Ring ausbilden, ausgewählt aus der Gruppe bestehend aus: -CH2-CH2-CH2-CH2-, -CH=CH-CH=CH-, -CH2-CH2-CH2-CH2-CH2-, -CH=CH-CH2-CH2-CH2-, -CH2-CH=CH-CH2-CH2-, -CH=CH-CH=CH-CH2-,-CH=CH-CH=CH-CH=.

3. Aromatische Alkensäurederivate der Formel (I) nach Anspruch 1 oder 2, wobei Q einen Rest 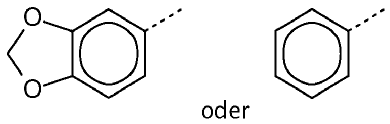 darstellt,
und entweder n einen Wert 0 und gleichzeitig m einen Wert 1 oder n einen Wert 1 und m gleichzeitig einen Wert 1 oder 2 darstellt, und wobei die resultierende(n) Doppelbindung(en) bevorzugt mit >50 %, bevorzugt > 80 %, besonders bevorzugt > 95% in der (*E*)-Konfiguration vorliegen, und
X = - OR¹ mit
R¹ = Wasserstoff, Methyl, Ethyl
oder - NR²R³ bedeutet,
und wobei
R² Wasserstoff ist
und gleichzeitig
R³ 2-Methyl-1-propyl, 2-Methyl-1-butyl
oder
R² und R³ zusammen einen gesättigten oder ungesättigten carbocyclischen Ring ausbilden, ausgewählt aus der Gruppe bestehend aus: -CH2-CH2-CH2-CH2-, -CH2-CH2-CH2-CH2-CH2-.

4. Aromatische Alkensäurederivate nach einem der Ansprüche 1 bis 3, wobei die aromatischen Alkensäurederivate ausgewählt sind aus der Gruppe bestehend aus:
(2*E*)-*N*-(Cinnamoyl)piperidine (Verbindung 1)
(2*E*)-3-(1,3-Benzodioxol-5-yl)-*N*-isobutyl-prop-2-enamid (*trans-*Fagaramid , Verbindung 2)
(2*E*)-3-(1,3-Benzodioxol-5-yl)-*N*-piperidinyl-prop-2-enamid (Ilepcimide, Verbindung 3) (2*E*)-5-(1,3-Benzodioxol-5-yl)-*N*-isobutyl-pent-2-enamid (Dihydropiperlonguminin,
Verbindung 4)
(2*E*,4*E*)-7-(1,3-Benzodioxol-5-yl)-*N*-isobutyl-hepta-2,4-dien-1-amid (Chingchengenamide A, Verbindung 5)
(*2*E,4*E*)-Methyl-7-(1,3-benzodioxol-5-yl)hepta-2,4-dienoat (Verbindung 6).

5. Aromamischung, umfassend
(a) mindestens ein aromatisches Alkensäurederivat der Formel (I) nach einem der Ansprüche 1 bis 4, und
(b) mindestens einen weiteren Geschmacksstoff.

6. Aromamischung nach Anspruch 5, **dadurch gekennzeichnet, dass** der weiterer Geschmacksstoff (b) für sich genommen mindestens einen der Geschmackseindrücke Umami, Kokumi oder salzig vermittelt oder in der Lage ist, unangenehme Geschmackseindrücke wie bitter, metallisch, kalkig, sauer oder adstringierend zu maskieren.

7. Aromamischung nach Anspruch 5 oder 6, wobei der weiterer Geschmacksstoff (b) ausgewählt ist aus der Gruppe, die gebildet wird von Mononatriumglutamat, freier Glutaminsäure, Nucleotiden oder deren pharmazeutisch akzeptablen Salzen, Strombinen, Theogallinen, Pyridin-Betain-Verbindungen, Glutaminsäureglycosiden, Äpfelsäureglycosiden, Glutathion-Derivaten, Lactisolen und Alkylpyridinen, Hesperetin, 4-Hydroxychalcone, Hydroxyflavanone, 3,7'-Dihydroxy-4'-methoxyflavan, Zimtsäureamiden, Phloretin, Rubemamin, Rubescenamin sowie Gemische daraus.

8. Aromamischung nach einem der Ansprüche 5 bis 7, wobei die Menge an Verbindungen der Formel (I) nach einem der Ansprüche 1 bis 4, im Bereich von 0,0001 Gew.% bis 95 Gew.-% in der Aromamischung vorliegt, bezogen auf die Gesamtmenge aller Verbindungen nach Formel (I) in der Aromamischung.

9. Verwendung von aromatischen Alkensäurederivate der Formel I nach einem der Ansprüche 1 bis 4 oder Aromamischungen nach einem der Ansprüche 5 bis 7 als Geschmackstoffe zur Vermittlung oder Erzielung eines selbstständigen oder Verstärkung eines vorhandenen Umami- oder Kokumi-artigen oder salzigen Geschmackeffekts.

10. Orale Zubereitungen, umfassend
(i) mindestens eine Aromamischung nach einem der Ansprüche 5 bis 8, oder
(ii) mindestens eine Verbindung der Formel (I) nach einem der Ansprüche 1 bis 4, und
(iii) mindestens einen weiteren zum Verzehr geeigneten Bestandteil ausgewählt aus der Gruppe die gebildet wird von Trägerstoffen, Aromastoffen, Fetten und Ölen, Emulgatoren, Antioxidantien, weitere Hilfs- und Zusatzstoffe, Natriumglutamat, Zucker und Salz.

11. Orale Zubereitungen nach Anspruch 10, wobei es sich bei den Zubereitungen um eine der Ernährung oder dem Genuss dienende gebrauchs- oder verzehrfertige Zubereitungen oder entsprechende Halbfertigprodukte handelt.

12. Orale Zubereitungen nach Anspruch 10 oder 11, wobei die Zubereitungen ausgewählt sind aus der Gruppe, die gebildet wird von Backwaren, Getränken, Instantgetränken, Fleischprodukten, gewürzten oder marinierten Fischprodukten, Eiern oder Eiprodukten Getreideprodukten, Milchprodukten Produkten aus Sojaprotein oder anderen Sojabohnen-Fraktionen, Gemüsezubereitungen, Knabberartikeln, Produkten auf Fett- und Ölbasis oder Emulsionen derselben, sonstigen Fertiggerichten, Suppen, Soßen, Gewürzen oder Gewürzzubereitungen sowie Aufstreuwürzungen.

13. Orale Zubereitungen nach einem der Ansprüche 10 bis 12, wobei die Verbindungen der Formel (I) in einer Menge von 5 ppm bis 500 ppm in einer Zubereitung enthalten sind, bezogen auf das Gesamtgewicht der Zubereitung.

14. Orale Zubereitungen nach einem der Ansprüche 10 bis 13, wobei die Zubereitungen
(i) einen reduzierten Gehalt an Natriumglutamat aufweisen oder völlig frei davon sind und/ oder
(ii) einen reduzierten Gehalt an Salz aufweisen oder völlig frei davon sind.
